# EUROPEAN PATENT APPLICATION

(11) **EP 4 755 907 A2**
(43) Date of publication of application: **10.06.2026**
(21) Application number: 26164712.7
(22) Date of filing: 01.11.2022
(51) Int. Cl.: C07K 14/705

(54) **PHARMACEUTICAL COMPOSITIONS OF HUMANIZED ANTI-CD40 ANTIBODIES AND USES THEREOF**

(30) Priority: 05.11.2021 US 202163276381 P; 24.02.2022 US 202263313583 P
(62) Divisional of application: 22890976.8
(71) Applicant: Kiniksa Pharmaceuticals, GmbH, 6302 Zug (CH)
(72) Inventor: PAOLINI, John F., Lexington, MA 02421 (US); KRANZ, James, Lexington, MA 02421 (US)
(74) Representative: Simmons & Simmons LLP (Munich)

(57) **Abstract**

The present disclosure relates to dosing regimens of pharmaceutical compositions of anti-CD40 antibodies, such as humanized anti-CD40 antibodies, and uses thereof in the treatment of rheumatoid arthritis.

## Description

### Sequence Listing

This application contains a Sequence Listing which has been filed electronically in Extensible Markup Language (XML) format and is hereby incorporated by reference in its entirety. Said XML copy, created on October 31, 2022, is named 51383-005WO3_Sequence_Listing_10_31_22.XML and is 20,949 bytes in size.

### Background

Suppression of the immune system, particularly the humoral immune system, is beneficial in organ transplantation and treatment of autoimmune disorders, such as rheumatoid arthritis.

One target for suppressing the immune system is the CD40/CD154 interaction. CD40 is expressed primarily on the surface of B lymphocytes and other antigen-presenting cells (APCs) such as dendritic cells and macrophages. CD154 is expressed primarily on the surface of T cells. The interaction between these two proteins is associated with B cell activation, which triggers cytokine expression as well as expression of cell surface markers including CD23, CD80, and CD86. Antibodies (e.g., humanized antibodies) that target the CD40/CD154 interaction have been developed.

There exists a need for therapeutic regimens that are effective for treating rheumatoid arthritis.

### Summary

A first aspect features a method of treating rheumatoid arthritis in a subject in need thereof by intravenously or subcutaneously administering, or by infusing, into the subject a pharmaceutical composition containing a humanized anti-CD40 antibody or antigen-binding fragment thereof with a heavy chain variable region having an amino acid sequence with at least 80% (e.g., at least 85%, 90%, 95%, 97%, 99%,or 100%) sequence identity to the amino acid sequence set forth in SEQ ID NO: 9 and a light chain variable region having an amino acid sequence with at least 80% (e.g., at least 85%, 90%, 95%, 97%, 99%,or 100%) sequence identity to the amino acid sequence set forth in SEQ ID NO: 10, in which the antibody or antigen-binding fragment thereof is administered at a dosage of from about 1 mg/kg to about 20 mg/kg, e.g., from about 1 mg/kg to about 10 mg/kg (e.g., from about 2 mg/kg to about 10 mg/kg, e.g., about 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg).

In some embodiments, the pharmaceutical composition administered to the subject includes a concentration of the antibody or antigen-biding fragment thereof of from of about 100 mg/ml to about 300 mg/mL and, optionally, in a volume of about 1.0 to 2.0 mL.

In some embodiments, the antibody or antigen-binding fragment thereof is administered at a concentration of about 200 mg/mL.

In some embodiments, a volume of about 0.5 mL to about 5.0 mL (e.g., 0.5 mL, 0.6 mL, 0.7 mL, 0.8 mL, 0.9 mL, 1.0 mL, 1.1 mL, 1.2 mL, 1.3 mL, 1.4 mL, 1.5 mL, 1.6 mL, 1.7 mL, 1.8 mL, 1.9 mL, 2.0 mL, 3.0 mL, 4.0 mL, or 5.0 mL) of the composition is administered to the subject. In some embodiments, a volume of about 1.0 mL is administered.

The antibody or antigen-binding fragment thereof can be administered at a dosage of from about 1 mg/kg to about 20 mg/kg, e.g., from about 1 mg/kg to about 10 mg/kg (e.g., from about 2 mg/kg to about 10 mg/kg, e.g., about 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg), e.g., once per week, once every two weeks, or once per month. In some embodiments, the composition is administered to the subject in a volume of about 0.1 mL to about 2.0 mL (e.g., 0.1 mL, 0.2 mL, 0.3 mL, 0.4 mL, 0.5 mL, 0.6 mL, 0.7 mL, 0.8 mL, 0.9 mL, 1.0 mL, 1.1 mL, 1.2 mL, 1.3 mL, 1.4 mL, 1.5 mL, 1.6 mL, 1.7 mL, 1.8 mL, 1.9 mL, or 2.0 mL, e.g., about 1.0 mL, and the antibody or antigen-binding fragment thereof is present in the composition at a concentration of from about 100 mg/mL to about 300 mg/mL (e.g., 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, 200 mg/mL, 210 mg/mL, 220 mg/mL, 230 mg/mL, 240 mg/mL, 250 mg/mL, 260 mg/mL, 270 mg/mL, 280 mg/mL, 290 mg/mL, or 300 mg/mL, e.g., about 200 mg/mL.

In some embodiments, the pharmaceutical composition includes, in addition to the antibody or antigen-binding fragment thereof (e.g., in a concentration of about 100-300 mg/mL and, optionally, in a volume of about 0.5 mL-3.0 mL), sodium acetate, arginine, glutamate, polysorbate 20. For example, the pharmaceutical composition includes sodium acetate in an amount of from about 5 mM to about 100 mM (e.g., about 10 mM to about 90 mM, e.g., about 25 mM to about 75 mM, e.g., about 40 mM to about 60 mM, such as, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM, in particular about 50 mM); arginine in an amount of from about 1 mM to about 200 mM (e.g., about 50 mM to about 150 mM, e.g., about 75 mM to about 125 mM, e.g., about 90 mM to about 110 mM, e.g., about 95 mM to about 105 mM, such as, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, in particular about 100 mM); glutamate in an amount of from about 1 mM to about 200 mM (e.g., about 50 mM to about 150 mM, e.g., about 75 mM to about 125 mM, e.g., about 90 mM to about 110 mM, e.g., about 95 mM to about 105 mM, such as, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, in particular about 100 mM); and polysorbate 20 in an amount of from about 0.001% to about 0.1% (e.g., about 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%, in particular about 0.01% or about 0.02%). The pharmaceutical composition may have a pH of about 5.0 to 6.0, such as a pH of 5.4.

In some embodiments, the pharmaceutical composition includes polysorbate 20, acetate, and one or more polar excipients. The polar excipient may be or may include, for example, a sugar, a polyol, or an amino acid. In some embodiments, the sugar is, for example, sucrose, trehalose, fructose, lactose, dextrose, or mannitol. In some embodiments, the polyol is, for example, polyethylene glycol or sorbitol. In some embodiments, the amino acid is one or more of alanine, arginine, aspartic acid, asparagine, carnitine, citrulline, ornithine, glycine, glutamic acid, glutamine, glycine, isoleucine, leucine, lysine, methionine, phenylalanine, proline, serine, threonine, tyrosine, and valine. The acetate may include, for example, sodium acetate (e.g., in salt form) or acetate in its ionic form.

In some embodiments, the pharmaceutical composition includes from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 45 mM, 50 mM, 60 mM, 70 mM, 80 mM, 90 mM, or 100 mM) acetate. In some embodiments, the pharmaceutical composition includes about 50 mM acetate.

In some embodiments, the pharmaceutical composition includes from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM) of the polar excipient. In some embodiments, the pharmaceutical composition includes about 50 mM of the polar excipient. In some embodiments, the pharmaceutical composition includes about 100 mM of the polar excipient. In some embodiments, the pharmaceutical composition includes about 150 mM of the polar excipient. In some embodiments, the pharmaceutical composition includes about 200 mM of the polar excipient.

In some embodiments, the pharmaceutical composition includes from about 1% (w/v) to about 10% (w/v) (e.g., about 2% (w/v) to about 8% (w/v), e.g., about 1% (w/v), 1.5 % (w/v), 2% (w/v), 2.5% (w/v), 3% (w/v), 3.5% (w/v), 4% (w/v), 4.5% (w/v), 5% (w/v), 5.5% (w/v), 6% (w/v), 6.5% (w/v), 7% (w/v), 7.5% (w/v), 8% (w/v), 8.5% (w/v), 9% (w/v), 9.5% (w/v), 10% (w/v)) of the polar excipient. In some embodiments, the pharmaceutical composition includes about 2.5% (w/v) of the polar excipient. In some embodiments, the pharmaceutical composition includes about 7% (w/v) of the polar excipient.

In some embodiments, the pharmaceutical composition includes from about 0.001% to about 0.1% (e.g., about 0.005% to 0.05%, e.g., about 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) polysorbate 20. In some embodiments, the pharmaceutical composition includes about 0.01% polysorbate 20. In some embodiments, the pharmaceutical composition includes about 0.02% polysorbate 20.

The composition may have a pH of from about 5.0 to about 8.0, e.g., about 5.4 to about 6.5; e.g., about 5.4 to about 5.75; e.g., 5.4 to about 5.6; e.g., 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0, e.g., about 5.4.

In some embodiments, the pharmaceutical composition includes about 50 mM sodium acetate, about 100 mM arginine, 100 mM glutamate, and about 0.02% polysorbate 20 and a pH of about 5.4. In some embodiments, the pharmaceutical composition includes about 50 mM acetate, about 50 mM arginine, 50 mM glutamate, and about 0.01% polysorbate 20. In some embodiments, the pharmaceutical composition includes about 50 mM acetate, about 50 mM arginine, 50 mM glutamate, and about 0.02% polysorbate 20. In some embodiments, the pharmaceutical composition includes about 50 mM acetate, about 100 mM arginine, 50 mM glutamate, and about 0.01% polysorbate 20. In some embodiments, the pharmaceutical composition includes about 50 mM acetate, about 100 mM arginine, 50 mM glutamate, and about 0.02% polysorbate 20.

In some embodiments, the composition is administered by intravenous or subcutaneous administration of by infusion. For example, the antibody or antigen-binding fragment thereof may be administered intravenously at a dosage of about 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg. In another example, the antibody or antigen-binding fragment thereof may be administered subcutaneously at a dosage of about 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg.

The composition may be administered, e.g., once per day, once per week, once every two weeks, once every three weeks, once per month, or once per year. In some embodiments, the composition is administered during a treatment regimen of at least one day, one week, two weeks, three weeks, one month, two months, three months, four months, five months, six months, one year, or longer, such as for the life of the subject. In one embodiment, the composition is administered to the subject until clinical remission is achieved or a minimum low disease activity is achieved.

In some embodiments, the antibody or antigen-binding fragment thereof is administered subcutaneously at a concentration of about 2 mg/kg, 5 mg/kg, or 10 mg/kg once every two weeks. Alternatively, the antibody or antigen-binding fragment thereof is administered subcutaneously at a concentration of about 2 mg/kg, 5 mg/kg, or 10 mg/kg once every three or four weeks. For example, the composition may be administered during a treatment regimen of about 12 weeks or longer (e.g., for six months, one year, two years, three years, four years, five years, six years, or more, such as for the life of the subject).

The method may further include administration of an immunosuppressant, a disease modifying anti-rheumatic drug (DMARD), or a Janus kinase (JA) inhibitor, e.g., within six months of the administration of the antibody or antigen-binding fragment thereof.

In some embodiments, the antibody or antigen-binding fragment thereof is administered to a subject with active rheumatoid arthritis. In certain embodiments, the antibody or antigen-binding fragment thereof is administered to a subject with active rheumatoid arthritis who has an inadequate response (IR) to or is intolerant to a JAK inhibitor (JAKi) or at least one disease-modifying anti-rheumatic drug (DMARD). In one embodiment, the DMARD is a conventional synthetic DMARDs (csDMARDs) (e.g., methotrexate, sulfasalazine, hydroxychloroquine, and leflunomide). In another embodiment, the DMARD is a biological DMARD (bDMARD; e.g., etanercept; adalimumab, infliximab, certolizumab, golimumab, abatacept, tocilizumab, sarilumab, and rituximab). In some embodiments the subject is classified as ACR RA functional Class 1-3.

The immunosuppressant may be selected from the group consisting of a calcineurin inhibitor, tacrolimus, an mTor inhibitor, fingolimod, myriocin, alemtuzumab, rituximab, an anti-CD4 monoclonal antibody, an anti-LFA1 monoclonal antibody, an antiLFA3 monoclonal antibody, an anti-CD45 antibody, an anti-CD19 antibody, monabatacept, belatacept, indolylASC; azathioprine, lymphocyte immune globulin and anti- thymocyte globulin [equine], mycophenolate mofetil, mycophenolate sodium, daclizumab, basiliximab, cyclophosphamide, prednisone, prednisolone, leflunomide, FK778, FK779, 15-deoxyspergualin, busulfan, fludarabine, methotrexate, 6-mercaptopurine, 15-deoxyspergualin, LF15-0195, bredinin, brequinar, and muromonab-CD3. In some embodiments, the calcineurin inhibitor is cyclosporin A or cyclosporine G; the mTor inhibitor is sirolimus, temsirolimus, zotarolimus, or everolimus; the anti-CD45 antibody is an anti-CD45RB antibody; or the immunosuppressant is belatacept. The antibody or antigen-binding fragment thereof and the immunosuppressant may be administered within one month of each other or within one week of each other.

The JAK inhibitor may be, for example, ruxolitinib, tofacitinib, oclacitinib, baricitinib, peficitinib, fedratinib, upadacitinib, filgotinib, or delgocitinib,

The DMARD may be, for example, abatacept, adalimumab, anakinra, apremilast, azathioprine, baricitinib, certolizumab pegol, chloroquine, ciclosporin (Cyclosporin A), D-penicillamine, etanercept, filgotinib, golimumab, gold salts (e.g., sodium aurothiomalate or auranofin), hydroxychloroquine, infliximab, leflunomide, methotrexate, minocycline, rituximab, sarilumab, secukinumab, sulfasalazine, tocilizumab, tofacitinib, or ustekinumab.

In some embodiments, the DMARD is a conventional synthetic DMARD.

In some embodiments, the subject has had an inadequate response, or is intolerant, to a JAK inhibitor or a DMARD.

In some embodiments, the method inhibits or reduces (e.g., by at least 10%, 20%, 30%, 40%, 50%, 60%, 70%, 80%, 90%, or 100%) one or more symptoms associated with RA. The one or more symptoms associated with RA may include, for example, inflamed (e.g., chronically inflamed) joints, cardiovascular disease, osteoporosis, interstitial lung disease, infection, cancer, tiredness, depression, mental difficulties, trouble working, inflammation of the synovial membrane, pain, finger deformity, osteoarthritis, ulnar deviation, boutonniere deformity, sawn neck deformity, Z-thumb (e.g., hyperextension of the interphalangeal joint, fixed flexion and subluxation of the metacarpophalangeal joint, hammer toe, and arthritis mutilans. The inhibition or reduction of a symptom may be measured with respect to a control subject (e.g., one who does not undergo a treatment regimen as described herein) or relative to the subject's baseline established prior to or after an earlier treatment, e.g., with a different therapeutic regimen. The reduction may include a reduction in frequency and/or intensity of symptoms.

In an embodiment, the methods include using an efficacy metric, such as the Disease Activity Score in 28 Joints (DAS28) or DAS28 C-Reactive Protein (DAS28-CRP), to monitor treatment efficacy for a subject with RA. If the DAS28 score is < 2.6, the subject may be determined to be in remission. A DAS28 score of less than or equal to 3.2 may indicate that the subject is in remission or has moderate or low disease activity and, thus, may require a reduced dosage of the therapeutic composition described herein (e.g., less than 10 mg/kg per dose), a reduced frequency of administration (e.g., a dose of the therapeutic composition administered once every week, once every two weeks, once every three weeks, or once every four weeks, or as needed to promote a reduction in symptoms exhibited during an acute flare-up or a reduction of a chronic symptom(s)), or both. If the DAS28 score is between 3.2 and 5.1, the subject may exhibit moderate or severe disease activity. Treatment for moderate or severe disease activity may involve administering a therapeutic composition described herein at a dose of about 10 mg/kg per dose or greater (e.g., about 20 mg/kg per dose). The dose may alternatively or in addition be administered at a greater frequency, such as, e.g., one or more times per week or two to five times every two to four weeks or until a symptom(s) of the RA in the subject diminishes (e.g., until the subject achieves a DAS28 score of <3.2).

In another embodiment, the efficacy metric used to assess treatment efficacy is the American College of Rheumatology (ACR) 20/50/70 assessment. The ACR20 is a composite measure defined as both improvement of 20% in the number of tender and number of swollen joints, and a 20% improvement in three of the following five criteria: patient global assessment, physician global assessment, functional ability measure [most often Health Assessment Questionnaire (HAQ)], visual analog pain scale, and erythrocyte sedimentation rate or C-reactive protein (CRP). The ACR50 and ACR70 metrics correspond to 50% and 70% improvement, respectively. The foregoing assessments may be used to guide dosing strategies, e.g., if the subject requires an increased or reduced dosage or frequency of administration of the antibody or antigen-binding fragment thereof in response to a particular efficacy metric (e.g., similar to the dosing regimen changes discussed above in conjunction with the DAS28 metric). For example, if the subject does not exhibit an ACR20, ACR50, or ACR70 score showing improvement in the tested measures, the subject may be administered an increased dosage of the therapeutic composition described herein (e.g., greater than 10 mg/kg per dose (e.g., 20 mg/kg per dose)), an increased frequency of administration (e.g., one or more times per week or two to five times every two to four weeks or until a symptom(s) of the RA in the subject diminishes), or both. Alternatively, if the subject exhibits an ACR20, ACR50, or ACR70 score showing improvement in the tested measures, the subject may be administered a reduced dosage of the therapeutic composition described herein, a reduced frequency of administration of the composition, or both (as is discussed above).

The methods described herein may include a treatment regimen that includes two phases of treatment. For example, a treatment regimen may contain an acute phase of treatment that includes, for example, increased dosages of the antibody or antigen-binding fragment thereof, increased frequency of dosing, or both. An acute phase of treatment may include, for example, intravenously or subcutaneously administering the antibody or antigen-binding fragment thereof at a dosage of from about 5 mg/kg to about 10 mg/kg (e.g., about 5 mg/kg, 6 mg/kg, 7 mg/kg, 8 mg/kg, 9 mg/kg, or 10 mg/kg), for example, once every two weeks, once every week, or twice every week, or more. The subject can then be monitored for a reduction (e.g., by at least 10%, 20%, 30%, 40%, 50%, 60%, 70%, 80%, 90%, or 100%) of one or more symptoms associated with RA. The inhibition or reduction of a symptom may be measured with respect to a control subject (e.g., a healthy subject or a different subject with RA that does not undergo a treatment regimen as described herein) or relative to the subject prior to the acute phase of treatment. The reduction may include a reduction in frequency and/or intensity of symptoms, e.g., as measured by an assessment as described herein. A reduction in frequency and/or amount of the antibody or antigen-biding fragment thereof may be determined based on an efficacy metric as described herein (e.g., DAS28, DAS28 CRP, or ACR 20/50/70 assessment). For example, if the subject achieves a DAS28 score of <3.2, the subject may be determined to require a reduced frequency or dosage of the antibody or antigen-biding fragment thereof.

After the acute phase of treatment, e.g., that results in reduction of one or more symptoms of RA or a reduced DAS28 score (e.g., of less than 2.6 or less than 3.2), the subject may then transition to a chronic dosing regimen that includes, for example, a reduced dosage of the antibody or antigen-binding fragment thereof, reduced frequency of dosing, or both, relative to the acute phase of treatment. For example, a chronic phase of treatment may include, for example, intravenously or subcutaneously administering the antibody or antigen-binding fragment thereof at a dosage of from about 1 mg/kg to about 5 mg/kg (e.g., about 1 mg/kg, 1.5 mg/kg, 2 mg/kg, 2.5 mg/kg, 3 mg/kg, 3.5 mg/kg, 4 mg/kg, 4.5 mg/kg, or 5 mg/kg), for example, once every four weeks, once every two weeks, or once every week.. Such a chronic dosing regimen may provide for a reduced overall dosage administered to the subject while still maintaining therapeutic efficacy.

The subject may transition from acute dosing to chronic dosing (e.g., when a reduction in one or more RA symptoms is observed) or from chronic dosing to acute dosing (e.g., when a flare-up of one or more RA symptoms is observed) one or more times during treatment with the anti-CD40 antibody or antigen-binding fragment thereof, e.g., as needed based on the observed RA symptom(s) in the subject.

In certain embodiments, the subject is a responder to the treatment with the anti-CD40 antibody or antigen-binding fragment thereof and is identified as having a statistically significant improvement in disease activity, wherein the disease activity is determined by one or more criteria selected from the group consisting of ACR20, ACR50, ACR70, DAS28, DAS28-CRP, Physician's Global Assessment (PhGA) of disease activity, Health Assessment Questionnaire-Disability Index (HAQ-DI), Short Form Health survey (SF-36) in the physical health composite score (PCS) and mental health composite score (MCS), SF-36 total composite score (TCS), Clinical Disease Activity Index (CDAI), SDAI (derived from CDAI plus DAS28-CRP, and a pain Visual Analog Scale (VAS). In a particular embodiment, a subject achieves a significant improvement in ACR20 response for KPL-404 vs. placebo by week 12 of the treatment. In another embodiment, a subject achieves a significant improvement in ACR20 response for KPL-404 vs. placebo by week 24 of the treatment. In one embodiment, a subject achieves a significant improvement in ACR20 response for KPL-404 vs. placebo by week 52 of the treatment. In a particular embodiment, a subject achieves a significant improvement in ACR50 response for KPL-404 vs. placebo by week 12 of the treatment. In another embodiment, a subject achieves a significant improvement in ACR50 response for KPL-404 vs. placebo by week 24 of the treatment. In one embodiment, a subject achieves a significant improvement in ACR50 response for KPL-404 vs. placebo by week 52 of the treatment.

In some embodiments, a 20% improvement in the American College of Rheumatology core set disease index (ACR20) is achieved after treatment.

In some embodiments, a 50% improvement in the American College of Rheumatology core set disease index (ACR50) is achieved after treatment.

In some embodiments, the ACR20 is achieved following a treatment regimen of about 1 week. In some embodiments, the ACR20 is achieved following a treatment regimen of about 24 weeks. In some embodiments, the ACR20 is achieved following a treatment regimen of about 52 weeks. In some embodiments, the ACR20 is achieved following a treatment regimen of about 1 week to about 52 weeks.

In some embodiments, the ACR50 is achieved following a treatment regimen of about 12 weeks. In some embodiments, the ACR50 is achieved following a treatment regimen of about 24 weeks. In some embodiments, the ACR50 is achieved following a treatment regimen of about 52 weeks. In some embodiments, the ACR50 is achieved following a treatment regimen of about 12 weeks to about 52 weeks.

In some embodiments, the disease activity score of 28 joints using C-reactive protein (DAS28-CRP) is achieved after treatment. In some embodiments, the DAS28-CRP is achieved following a treatment regimen of about 12 weeks. In some embodiments, the DAS28-CRP is achieved following a treatment regimen of about 24 weeks. In some embodiments, the DAS28-CRP is achieved following a treatment regimen of about 52 weeks. In some embodiments, the DAS28-CRP is achieved following a treatment regimen of about 12 weeks to about 52 weeks.

In some embodiments, after treatment, the subject may achieve an improvement in a disease activity determined by a criteria selected from the group consisting of ACR20, ACR50, ACR70, DAS28, DAS28-CRP, Physician's Global Assessment (PhGA) of disease activity, Health Assessment Questionnaire-Disability Index (HAQ-DI), Short Form Health survey (SF-36) in the physical health composite score (PCS) and mental health composite score (MCS), SF-36 total composite score (TCS), Clinical Disease Activity Index (CDAI), Simple Disease Activity Index (SDAI), and a pain Visual Analog Scale (VAS), and combinations thereof.

### Definitions

As used herein, the term "about" means ±10% of the recited value.

### Brief Description of the Drawings

**FIG. 1** is a schematic drawing showing an overall scheme for a clinical study involving administration of a pharmaceutical composition of the disclosure.
**FIG. 2** is a schematic drawing showing a clinical trial scheme for administration of a pharmaceutical composition of the disclosure.

### Detailed Description

The disclosure features methods of treating, reducing, or inhibiting rheumatoid arthritis in a subject in need thereof by administering a pharmaceutical composition containing an anti-CD40 antibody or antigen binding fragment thereof in an amount of from about 1 mg/kg to about 10 mg/kg (e.g., about 2 mg/kg, 5 mg/kg, or 10 mg/kg). The pharmaceutical composition contains anti-CD40 antibodies or antigen-binding fragments thereof that can block the ability of CD40 to bind CD154 and do so without activating the cell expressing CD40 (e.g., a B cell). Anti-CD40 treatment can also attenuate T-cell function, such as T-cell cytotoxicity (e.g., via CD40 blockade on antigen presenting cells), which can be therapeutic against rheumatoid arthritis, in particular in a subject experiencing an inadequate response to or that is intolerant to a JAK inhibitor (JAKi) or at least one disease-modifying anti-rheumatic drug (DMARD).

In general, the antibody or antigen-binding fragment thereof is derived from the murine 2C10 antibody and humanized variants thereof, e.g., as described in PCT Pub. Nos. WO 2012/125569 and WO 2017/040932, which are herein incorporated by reference in their entirety. The methods and the pharmaceutical compositions used therein are described in more detail below.

### Antibodies and Antigen-Binding Fragments Thereof

The methods described herein include administration of an antibody or antigen-binding fragment thereof, e.g., derived from the murine 2C10 antibody. The heavy chain variable regions, light chain variable regions, and CDRs of certain anti-CD40 antibodies described herein are shown in **Table 1.**

**Table 1: 2C10 and KPL-404 antibody sequences**

| **Name** | **Chain, Region** | **Sequence** | **SEQ ID NO:** |
|---|---|---|---|
| 2C10 (Murine antibody) | Heavy chain, variable region | QVQLQQSGAELAKPGASVKMSCKASGYTFTNYWMH WVKQRPGQGLEWIGYINPSNDYTKYNQKFKDKATLTA DKSSNTAYMQLGSLTSEDSAVYYCARQGFPYWGQG TLVTVSA | 1 |
| 2C10 | Light chain, variable region | QIVLTQSPAIMSASPGEKVTMTCSASSSVSYMHWYHQ RSGTSPKRWIYDTSKLASGVPARFSGSGSGTSYSLTI SSMEAEDAATYYCHQLSSDPFTFGSGTKLEIK | 2 |
| 2C10 and KPL-404 | Heavy chain, CDR1 | YTFTNYWMH | 3 |
| 2C10 and KPL-404 | Heavy chain, CDR2 | YINPSNDYTKYNQKFKD | 4 |
| 2C10 and KPL-404 | Heavy chain, CDR3 | QGFPY | 5 |
| 2C10 and KPL-404 | Light chain, CDR1 | SASSSVSYMH | 6 |
| 2C10 and KPL-404 | Light chain, CDR2 | DTSKLAS | 7 |
| 2C10 and KPL-404 | Light chain, CDR3 | HQLSSDPFT | 8 |
| KPL-404 | Heavy chain, variable region | QVQLVQSGAEVKKPGASVKVSCKASG YTFTNYWMH WVRQAPGQRLEWIG YINPSNDYTKYNQKFKD RATLTADKSANTAYM ELSSLRSEDTAVYYCAR QGFPY WGQGTLVTVSS | 9 |
| KPL-404 | Light chain, variable region | EIVLTQSPATLSLSPGERATLSC SASSSVSYMH WYQQKPGQAPRRWIY DTSKLAS GVPARFSGSGSGTDYTLTISSLEPEDFAVYYC HQLSSDPFT FGGGTKVEIK | 10 |
| KPL-404 | Heavy chain (with IgG4 constant domain) | QVQLVQSGAEVKKPGASVKVSCKASGYTFTNYWMH WVRQAPGQRLEWIGYINPSNDYTKYNQKFKDRATLTA DKSANTAYMELSSLRSEDTAVYYCARQGFPYWGQGT LVTVSSASTKGPSVFPLAPCSRSTSESTAALGCLVKD YFPEPVTVSWNSGALTSGVHTFPAVLQSSGLYSLSSV VTVPSSSLGTKTYTCNVDHKPSNTKVDKRVESKYGPP CPPCPAPEFLGGPSVFLFPPKPKDTLMISRTPEVTCVV VDVSQEDPEVQFNWYVDGVEVHNAKTKPREEQFNST YRVVSVLTVLHQDWLNGKEYKCKVSNKGLPSSIEKTIS KAKGQPREPQVYTLPPSQEEMTKNQVSLTCLVKGFY PSDIAVEWESNGQPENNYKTTPPVLDSDGSFFLYSRL TVDKSRWQEGNVFSCSVMHEALHNHYTQKSLSLSPG K | 11 |
| KPL-404 | Light chain (with kappa constant domain) | EIVLTQSPATLSLSPGERATLSCSASSSVSYMHWYQQ KPGQAPRRWIYDTSKLASGVPARFSGSGSGTDYTLTI SSLEPEDFAVYYCHQLSSDPFTFGGGTKVEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC | 12 |
| 2C10_h1 | Heavy chain, variable region | QVQLVQSGAEVKKPGASVKVSCKASGYTFTNYWMH WVRQAPGQRLEWMGYINPSNDYTKYNQKFKDRVTIT RDTSASTAYMELSSLRSEDTAVYYCAR QGFPYWGQGTLVTVSS | 13 |
| 2C10_h2 | Heavy chain, variable region | QVQLVQSGAEVKKPGASVKVSCKASGYTFTNYWMH WVRQAPGQRLEWMGYINPSNDYTKYNQKFKDRVTIT ADKSASTAYMELSSLRSEDTAVYYCARQGFPYWGQG TLVTVSS | 14 |
| 2C10_I1 | Light chain, variable region | EIVLTQSPATLSLSPGERATLSCSASSSVSYMHWYQQ KPGQAPRLLIYDTSKLASGIPARFSGSGSGTDFTLTISS LEPEDFAVYYCHQLSSDPFTFGGGTKVEIK | 15 |
| 2C10HP | Heavy chain, variable region | QVQLVQSGAEVKKPGASVKVSCKASGYTFTNYWMH WVRQAPGQRLEWIGYINPSNDYTKYNQKFKDRATLTA DKSANTAYMELSSLRSEDTAVYYCARQGFPYWGQGT LVTVSS | 16 |
| 2C10HB1 | Heavy chain, variable region | QVQLVQSGAEVKKPGASVKVSCKASGYTFTNYWMH WVRQAPGQRLEWIGYINPSNDYTKYNQKFKDRATLTA DTSTNTAYMELSSLRSEDTAVYYCARQGFPYWGQGT LVTVSS | 17 |
| 2C10HB2 | Heavy chain, variable region | QVQLVQSGAEVKKPGSSVKVSCKASGYTFTNYWMH WVRQAPGQGLEWIGYINPSNDYTKYNQKFKDKATITA DESTNTAYMELSSLRSEDTAVYYCARQGFPYWGQGT LVTVSS | 18 |
| 2C10KP | Light chain, variable region | EIVLTQSPATLSLSPGERATLSCSASSSVSYMHWYQQ KPGQAPRRWIYDTSKLASGVPARFSGSGSGTDYTLTI SSLEPEDFAVYYCHQLSSDPFTFGGGTKVEIK | 19 |
| 2C10KB1 | Light chain, variable region | DIQMTQSPSTLSASVGDRVTITCSASSSVSYMHWYQQ KPGKAPKLLIYDTSKLASGVPARFSGSGSGTEFTLTIS SLQPDDFATYYCHQLSSDPFTFGQGTKVEVK | 20 |
| 2C10KB2 | Light chain, variable region | EIVLTQSPATLSLSPGERATLSCSASSSVSYMHWYQQ KPGQAPRLLIYDTSKLASGIPARFSGSGSGTDFTLTISS LEPEDFAVYYCHQLSSDPFTFGQGTKLEIK | 21 |

In certain embodiments, the antibody or antigen-binding fragment thereof includes a heavy chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to the heavy chain variable region amino acid sequence as set forth in SEQ ID NO: 9.

In certain embodiments, the antibody or antigen-binding fragment thereof includes a light chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to a light chain variable region amino acid sequence as set forth in SEQ ID NO: 10.

In certain embodiments, the antibodies or antigen-binding fragments thereof include both a heavy chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to a heavy chain variable region amino acid sequence as set forth in SEQ ID NO: 9, and a light chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to a variable light chain amino acid sequence as set forth in SEQ ID NO: 10.

In certain embodiments, the antibodies or antigen-binding fragments thereof include a heavy chain region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to a heavy chain with the amino acid sequence as set forth in SEQ ID NO: 11, and a light chain region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to the light chain amino acid sequence as set forth in SEQ ID NO: 12.

In certain embodiments, the antibodies or antigen-binding fragments thereof include a heavy chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% sequence identity to a heavy chain variable region amino acid sequence as set forth in any one of SEQ ID NOs: 13, 14, 16, 17, or 18, and a light chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% sequence identity to a variable light chain amino acid sequence as set forth in any one of SEQ ID NOs: 15, 19, 20, or 21.

In certain embodiments, a heavy chain variable region of the antibody or antigen-binding fragment thereof includes complementarity determining regions (CDRs) that are at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to the CDRs of a heavy chain variable region of the KPL-404 antibody (CDR1, CDR2 and CDR3 as set forth in SEQ ID NOs: 3, 4, 5, respectively).

In certain embodiments, the light chain variable region of the antibody or antigen-binding fragment thereof includes CDRs that are at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to the CDRs of a light chain variable region of the KPL-404 antibody (CDR1, CDR2 and CDR3 as set forth in SEQ ID NOs: 6, 7, 8, respectively).

In certain embodiments, the heavy chain includes the CDRs as set forth in SEQ ID NOs: 3-5, respectively, and the light chain includes the CDRs as set forth in SEQ ID NOs: 6-8, respectively.

In certain embodiments, the heavy chain has at least 80% (e.g., at least 85%, 90%, 95%, 97%, 99%, or 100%) sequence identity to SEQ ID NO: 9 and the CDRs set forth in SEQ ID NOs: 3-5, respectively, and the light chain has at least 80% (e.g., at least 85%, 90%, 95%, 97%, 99%, or 100%) sequence identity to SEQ ID NO: 10 and the CDRs set forth in SEQ ID NOs: 6-8, respectively.

Also within the scope of the disclosure are antibodies or antigen-binding fragments thereof in which specific amino acids have been substituted, deleted, or added. These alternations do not have a substantial effect on the peptide's biological properties such as binding activity. For example, antibodies may have amino acid substitutions in the framework region, such as to improve binding to the antigen. In another example, a selected, small number of acceptor framework residues can be replaced by the corresponding donor amino acids. The donor framework can be a mature or germline human antibody framework sequence or a consensus sequence. Guidance concerning how to make phenotypically silent amino acid substitutions is provided in, e.g., Bowie et al. (Science, 247: 1306-1310, 1990), Cunningham et al. (Science, 244: 1081-1085, 1989), Ausubel (ed.) (Current Protocols in Molecular Biology, John Wiley and Sons, Inc., 1994), T. Maniatis, E. F. Fritsch and J. Sambrook (Molecular Cloning: A Laboratory Manual, Cold Spring Harbor laboratory, Cold Spring Harbor, N.Y., 1989), Pearson (Methods Mol. Biol. 243:307-31, 1994), and Gonnet et al. (Science 256:1443-45, 1992); each of which is incorporated herein by reference.

The polypeptides described herein may be a functionally active variant of the antibodies or antigen-binding fragments thereof disclosed herein, e.g., with less than about 30%, about 25%, about 20%, about 15%, about 10%, about 5% or about 1% amino acid residues substituted or deleted but that retain essentially the same immunological properties including, but not limited to, binding to CD40.

In some embodiments, the dissociation constant (K_{D}) of the antibody or antigen-binding fragment thereof is less than about 1 x 10⁻⁸, e.g., less than about 1 x 10⁻⁹.

The antibodies or antigen-binding fragments thereof may also include one or more analogs of an amino acid (including, for example, non-naturally occurring amino acids, amino acids which only occur naturally in an unrelated biological system, modified amino acids from mammalian systems etc.), polypeptides with substituted linkages, as well as other modifications known in the art.

### Indications

The methods described herein include administration of a pharmaceutical composition containing an antibody or antigen-binding fragment as described herein to a subject with rheumatoid arthritis in an *in vivo* protocol (e.g., therapeutic or prophylactic) for the treatment of, or to reduce, inhibit, or mitigate one or more symptoms of, rheumatoid arthritis. The anti-CD40 antibodies or antigen-binding fragments thereof can be administered to treat the rheumatoid arthritis or to treat or reduce the likelihood of developing one or more symptoms of rheumatoid arthritis.

Rheumatoid arthritis (RA) is a long-term autoimmune disorder that primarily affects joints but may also affect other organs. RA is most often characterized by inflamed (e.g., chronically inflamed) joints. RA may also include or be associated with other secondary problems, including cardiovascular disease, osteoporosis, interstitial lung disease, infection, cancer, tiredness, depression, mental difficulties, and trouble working.

RA of the joints involves inflammation of the synovial membrane. Symptoms include, for example, swollen, tender, warm, and stiff joints and pain associated therewith. Fingers may suffer from deformity depending on which joints are involved, often resulting in osteoarthritis, ulnar deviation, boutonniere deformity, sawn neck deformity, Z-thumb (e.g., hyperextension of the interphalangeal joint, fixed flexion and subluxation of the metacarpophalangeal joint), hammer toe, and arthritis mutilans.

RA of the skin may result in an inflammatory reaction known as necrotizing granuloma, which results in nodule formation, including a central area of fibrinoid necrosis that may be fissured around an affected synovial space. Other skin issues include vasculitis, pyoderma gangrenosum, Sweet's syndrome, drug reactions, erythema nodosum, lobe panniculitis, atrophy of finger skin, palmar erythema, and skin fragility, and Diffuse alopecia areata.

Other sequelae associated with RA include, for example, lung fibrosis, exudative pleural effusions, atherosclerosis, myocardial infarction and stroke, pericarditis, endocarditis, left ventricular failure, valvulitis, fibrosis, anemia, renal amyloidosis, episcleritis, scleritis, keratoconjunctivitis sicca, keratitis, peripheral neuropathy, mononeuritis multiplex, carpal tunnel syndrome, atlanto-axial subluxation, local osteoporosis, lymphoma, and periodontitis.

Subjects with RA may be classified based on the severity of pervasiveness of the disease. For example, subjects may be stratified according to the number of joints involved (e.g., 1 large joint, 2-10 large joints, 1-3 small joints, 4-10 small joints, or more than 10 joints).

In some embodiments, a subject with RA may be classified as difficult to treat (D2T RA), in which the subject exhibits persistence of signs and symptoms, drug resistance, does not respond to two or more biological treatments, and does not respond to anti-rheumatic drugs with different mechanism of action.

In some embodiments, a subject is classified as ACR RA functional Class 1-3 using the 2010 American College of Rheumatology (ACR)/European Union League Against Rheumatism (EULAR) classification criteria for RA.

Standard-of-care treatment includes early therapy with disease-modifying anti-rheumatic drug (DMARDs), including conventional synthetic DMARDs (csDMARDs) (e.g., methotrexate, sulfasalazine, hydroxychloroquine, and leflunomide). Targeted biological DMARDs (bDMARDs) have advanced the treatment paradigm for RA, in which therapy is optimized until clinical remission or a minimum low disease activity is achieved. Despite a wide therapeutic armamentarium of bDMARDs, however, a blockade of any single cytokine or cellular subset has not shown control of disease in all RA subjects. In some embodiments, the antibody or antigen-binding fragment thereof is administered to a subject with active rheumatoid arthritis. In certain embodiments, the antibody or antigen-binding fragment thereof is administered to a subject with active rheumatoid arthritis who has an inadequate response to or is intolerant to a JAK inhibitor (JAKi-IR) or at least one disease-modifying anti-rheumatic drug (DMARD-IR). In one embodiment, the DMARD is a conventional synthetic DMARDs (csDMARDs) (e.g., methotrexate, sulfasalazine, hydroxychloroquine, and leflunomide). In another embodiment, the DMARD is a biological DMARD (e.g., etanercept; adalimumab, infliximab, certolizumab, golimumab, abatacept, tocilizumab, sarilumab, and rituximab).

In some embodiments, the subject has had an inadequate response, or is intolerant, to a JAK inhibitor or a DMARD.

The methods described herein may be used to treat the RA or, e.g., to reduce the occurrence or severity of one or more symptoms or associated sequelae of RA as described herein, e.g., as compared to a subject that is not treated with the anti-CD40 antibody or antigen-binding fragment thereof. For example, the methods may reduce the length of time during which a subject experiences one or more symptoms or associated sequelae of RA (e.g., by one or more days, weeks, or months). The methods described herein may be used to treat any classification or severity of RA (e.g., D2T RA) as described herein.

The methods described herein may inhibit or reduce (e.g., by at least 10%, 20%, 30%, 40%, 50%, 60%, 70%, 80%, 90%, or 100%) one or more symptoms associated with RA. The inhibition or reduction of a symptom may be measured with respect to a control subject (e.g., a healthy subject or a different subject with RA that does not undergo a treatment regimen as described herein) or relative to the subject prior to or after an earlier treatment, e.g., with a different therapeutic regimen, e.g., an unsuccessful therapeutic regimen. The reduction may include a reduction in frequency and/or intensity of symptoms.

The methods described herein may be used in combination with immunosuppression (e.g., by coadministration with an immunosuppressive agent).

### Pharmaceutical compositions

Featured are methods of treating, reducing, or inhibiting rheumatoid arthritis in a subject in need thereof, as described herein, by administration of a pharmaceutical composition containing an antibody or antigen-binding fragment thereof as described herein (e.g., an antibody or antigen-binding fragment thereof containing a heavy chain variable region having at least 80%, 85%, 90%, 95%, 97%, 99%, or 100% sequence identity to SEQ ID NO: 9 and a light chain variable region having at least 80%, 85%, 90%, 95%, 97%, 99%, or 100% sequence identity to SEQ ID NO: 10, formulated together with a pharmaceutically acceptable carrier). The antibody or antigen-binding fragment thereof can be formulated in a pharmaceutical composition in a concentration of about 50 to about 300 mg/mL, and optionally, in a volume of about 0.5 mL to about 2.0 mL (e.g., about 1.0 mL). The composition can be administered to the subject prior to the development of symptoms of RA, prior to the diagnosis of RA (e.g., in a subject predisposed to develop RA), or after the development of one or more symptoms of RA or a diagnosis of RA.

The antibody or antigen-binding fragment thereof (e.g., KPL-404) may be incorporated into a pharmaceutical composition (e.g., in a concentration of about 50 mg/mL to about 300 mg/mL, such as about 100 mg/mL or about 200 mg/mL) polysorbate 20, acetate, and one or more polar excipients. The polar excipient may be or may include, for example, a sugar, a polyol, or an amino acid. In some embodiments, the sugar is, for example, sucrose, trehalose, fructose, lactose, dextrose, or mannitol. In some embodiments, the polyol is, for example, polyethylene glycol or sorbitol. In some embodiments, the amino acid is one or more of alanine, arginine, aspartic acid, asparagine, carnitine, citrulline, ornithine, glycine, glutamic acid, glutamine, glycine, isoleucine, leucine, lysine, methionine, phenylalanine, proline, serine, threonine, tyrosine, and valine. The acetate may include, for example, sodium acetate (e.g., in salt form) or acetate in its ionic form. In some embodiments, the antibody or antigen-binding fragment. In some embodiments, the polar excipient is, for example sucrose, arginine, glutamate, sorbitol, or a combination thereof. In some embodiments, the polar excipient is sucrose. In some embodiments the polar excipient is arginine. In some embodiments the polar excipient is glutamate. In some embodiments, the polar excipient is a mixture of arginine and glutamate. In some embodiments, the polar excipient is sorbitol.

The pharmaceutical composition can include from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM, e.g., about 50 mM acetate, from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, e.g., about 100 mM), or from about 1% (w/v) to about 10% (w/v) (e.g., about 2% (w/v) to about 8% (w/v), e.g., about 1% (w/v), 1.5 % (w/v), 2% (w/v), 2.5% (w/v), 3% (w/v), 3.5% (w/v), 4% (w/v), 4.5% (w/v), 5% (w/v), 5.5% (w/v), 6% (w/v), 6.5% (w/v), 7% (w/v), 7.5% (w/v), 8% (w/v), 8.5% (w/v), 9% (w/v), 9.5% (w/v), 10% (w/v), e.g., about 2.5% (w/v) or about 7% (w/v) of the polar excipient, and from about 0.001% to about 0.1% (e.g., 0.001%, about 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%, e.g., about 0.01% or about 0.02%) polysorbate 20. The pharmaceutical composition may have a pH of from about 5.0 to about 8.0, e.g., about 5.4 to about 6.5; e.g., about 5.4 to about 5.75; e.g., 5.4 to about 5.6; e.g., 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0. For example, the pH may be about 5.4.

In some embodiments, the antibody or antigen-binding fragment (e.g., KPL-404) may be incorporated into a pharmaceutical composition (e.g., in a concentration of about 50 mg/mL to about 300 mg/mL, such as about 100 mg/mL or about 200 mg/mL) containing 50 mM acetate, 100 mM arginine, 100 mM glutamate, and 0.01% polysorbate 20, and a pH of 5.4. The antibody or antigen-binding fragment thereof (e.g., KPL-404) may be incorporated into a pharmaceutical composition (e.g., in a concentration of about 50 mg/mL to about 300 mg/mL, such as about 100 mg/mL or about 200 mg/mL) containing 50 mM acetate, 100 mM arginine, 100 mM glutamate, and 0.02% polysorbate 20, and a pH of 5.4. A pharmaceutical composition of the disclosure may also include modifications relative to this formulation. For example, the pharmaceutical composition can include polysorbate 20, sodium acetate, arginine, and glutamate in amounts that differ from the above amounts. For example, the pharmaceutical composition can include from about 5 mM to about 100 mM (e.g., about 10 mM, 20 mM, 30 mM, 40 mM, 50 mM, 60 mM, 70 mM, 80 mM, 90 mM, or 100 mM) sodium acetate, such as about 50 mM sodium acetate. The pharmaceutical composition can include from about 1 mM to about 200 mM (e.g., about 1 mM, 2 mM, 3 mM, 4 mM. 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM) arginine, such as about 100 mM arginine. The pharmaceutical composition can include from about 1 mM to about 200 mM (e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM) glutamate, such as about 100 mM glutamate. The pharmaceutical composition can include from about 0.001% to about 0.1% (e.g., 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) polysorbate 20, such as about 0.02% polysorbate 20. In some embodiments, the pharmaceutical composition includes about 50 mM acetate, about 100 mM arginine, 100 mM glutamate, and about 0.02% polysorbate 20. In some embodiments, the pharmaceutical composition includes about 50 mM acetate, about 50 mM arginine, 50 mM glutamate, and about 0.01% polysorbate 20. In some embodiments, the pharmaceutical composition includes about 50 mM acetate, about 50 mM arginine, 50 mM glutamate, and about 0.02% polysorbate 20. In some embodiments, the pharmaceutical composition includes about 50 mM acetate, about 100 mM arginine, 50 mM glutamate, and about 0.02% polysorbate 20. In some embodiments, the pharmaceutical composition includes about 50 mM acetate, about 100 mM arginine, 50 mM glutamate, and about 0.02% polysorbate 20.

For example, the pharmaceutical composition can include from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM, e.g., about 50 mM) sodium acetate, from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, e.g., about 100 mM) arginine, from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, e.g., about 100 mM) glutamate, and from about 0.001% to about 0.1% (e.g., 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%, e.g., about 0.02%) polysorbate 20. The pharmaceutical composition may have a pH of from about 5.0 to about 6.0. For example, the pH may be about 5.4.

The pharmaceutical composition can include, e.g., an amount of an anti-CD40 antibody or antigen-binding fragment thereof described herein (e.g., KPL-404), such as, e.g., about 50 mg/mL to about 300 mg/mL).

The antibody or antigen-binding fragment thereof (e.g., KPL-404) may also be incorporated into a pharmaceutical composition (e.g., in a concentration of about 100 mg/mL to about 300 mg/mL, such as 200 mg/mL) containing about 50 mM acetate, about 7% (w/v) sucrose, and about 0.01% polysorbate 20, in which the pharmaceutical composition has a pH of about 5.4.

The antibody or antigen-binding fragment thereof (e.g., KPL-404) may also be incorporated into a pharmaceutical composition (e.g., in a concentration of about 50 mg/mL to about 300 mg/mL, such as about 100 mg/mL or about 200 mg/mL) containing about 50 mM acetate, about 2.5% (w/v) sorbitol, about 70 mM NaCl, and about 0.02% polysorbate 20, in which the pharmaceutical composition has a pH of about 5.4.

The antibody or antigen-binding fragment thereof may be provided in a container including a pharmaceutical composition as described herein. Suitable containers include, for example, bottles, vials, syringes, and test tubes. The containers may be formed from a variety of materials such as glass or plastic. The container holds a pharmaceutical composition as described herein, e.g., that is effective for treating the condition, and may have a sterile access port. For example, the container may be an intravenous solution bag or a vial having a stopper pierceable by a hypodermic injection needle. The label on or associated with the container indicates that the composition is used for treating the condition of choice. The kit may further include a second container with a pharmaceutically acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use.

The antibody or antigen-binding fragment thereof may be present in the container at a concentration of from about 50 mg/mL to about 300 mg/mL (e.g., 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, 200 mg/mL, 210 mg/mL, 220 mg/mL, 230 mg/mL, 240 mg/mL, 250 mg/mL, 260 mg/mL, 270 mg/mL, 280 mg/mL, 290 mg/mL, or 300 mg/mL), such as at a concentration of about 100 mg/mL or about 200 mg/mL.

The composition may be formulated in a single use vial with about 1.0 mL or 2.0 mL extractable volume. The composition can be formulated in a volume of about 0.1 mL to about 2.0 mL (e.g., 0.1 mL, 0.2 mL, 0.3 mL, 0.4 mL, 0.5 mL, 0.6 mL, 0.7 mL, 0.8 mL, 0.9 mL, 1.0 mL, 1.1 mL, 1.2 mL, 1.3 mL, 1.4 mL, 1.5 mL, 1.6 mL, 1.7 mL, 1.8 mL, 1.9 mL, or 2.0 mL), such as a volume of about 1.0 mL.

### Routes of Administration and Methods of Treatment

A composition as described herein can be administered by a variety of methods known in the art. As will be appreciated by the skilled artisan, the route and/or mode of administration will vary depending upon the desired results. Administration may be parenteral, intravenous, intrathecal, subcutaneous, oral, topical, local, intramuscular, intradermal, transdermal, subdermal, rectal, spinal, or epidermal. Intravenous delivery by continuous infusion is one exemplary method for administering the present antibodies or antigen-binding fragments thereof.

The composition described herein may include a pharmaceutically acceptable diluent. Pharmaceutically acceptable diluents include, for example, saline and aqueous buffer solutions.

Parenteral administration can include modes of administration other than enteral and topical administration, usually by injection, and include, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrastemal injection and infusion.

The methods described herein may include administration of the pharmaceutical composition by infusion, intravenously, or subcutaneously. The methods may include administering the antibody or antigen-binding fragment thereof at a dosage of from about 1 mg/kg to about 20 mg/kg, e.g., from about 1 mg/kg to about 10 mg/kg (e.g., from about 2 mg/kg to about 10 mg/kg, e.g., about 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg).

For example, the antibody or antigen-binding fragment thereof may be administered intravenously at a dosage of about 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg.

In another example, the antibody or antigen-binding fragment thereof may be administered subcutaneously at a dosage of about 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg.

The composition may be administered, e.g., once per day, once per week, once every two weeks, once every three weeks, once per month, or once per year. In some embodiments, the composition is administered during a treatment regimen of at least one day, one week, two weeks, three weeks, one month, two months, three months, four months, five months, six months, one year, or longer, for example, for the life of the subject. In one embodiment, the composition is administered to the subject until clinical remission is achieved or a minimum low disease activity is achieved.

In one example, the antibody or antigen-binding fragment thereof is administered subcutaneously at a concentration of about 2 mg/kg, about 5 mg/kg, or about 10 mg/kg, e.g., once every two weeks. For example, the composition may be administered during a treatment regimen of about 12 weeks or longer, for example, for the life of the subject.

The methods described herein may include using an efficacy metric, such as the Disease Activity Score in 28 Joints (DAS28) or DAS28 C-Reactive Protein (DAS28-CRP), e.g., as described in Hansen et al. J. Clin. Rheumatol. 23:102-106, 2017, which is hereby incorporated by reference in its entirety. For example, the DAS28 or DAS28-CRP score can be used to monitor treatment efficacy for a subject with RA. If the DAS28 score is < 2.6, the subject may be determined to be in remission. A DAS28 score of less than or equal to 3.2 may indicate that the subject is in remission or has moderate or low disease activity and, thus, may require a reduced dosage of the therapeutic composition described herein (e.g., less than 10 mg/kg per dose, e.g., 5 mg/kg), a reduced frequency of administration (e.g., a dose of the therapeutic composition may be administered once every week, once every two weeks, once every three weeks, or once every four weeks, or once every other month, or as needed to promote a reduction in symptoms exhibited during an acute flare-up or a reduction of a chronic symptom(s)), or both. If the DAS28 score is between 3.2 and 5.1, the subject may exhibit moderate or severe disease activity. Treatment for moderate or severe disease activity may involve administering a therapeutic composition described herein at a dose of about 10 mg/kg per dose or greater (e.g., about 20 mg/kg per dose). The dose may alternatively or in addition be administered at a greater frequency, such as, e.g., one or more times per week, once every two weeks, or two to five times every two to four weeks or until a symptom(s) of the RA in the subject diminishes (e.g., until the subject achieves a DAS28 score of <3.2).

Another metric that may be used to assess treatment efficacy is the American College of Rheumatology (ACR) 20/50/70 assessment. A 20% improvement in the American College of Rheumatology core set disease index (ACR20) is a composite measure defined as both improvement of 20% in the number of tender and number of swollen joints, and a 20% improvement in three of the following five criteria: patient global assessment, physician global assessment, functional ability measure [most often Health Assessment Questionnaire (HAQ)], visual analog pain scale, and erythrocyte sedimentation rate or C-reactive protein (CRP). The ACR50 and ACR70 metrics correspond to 50% and 70% improvement in the American College of Rheumatology core set disease index, respectively. These assessments are described, for example, in US 2022/0025035, which is herein incorporated by reference in its entirety. The foregoing assessments may be used to guide dosing strategies, e.g., if the subject requires an increased or reduced dosage or frequency of administration of the antibody or antigen-binding fragment thereof in response to a particular efficacy metric (e.g., similar to the dosing regimen changes discussed above in conjunction with the DAS28 metric).

The methods described herein may include a treatment regimen that includes two phases of treatment. For example, a treatment regimen may contain an acute phase of treatment that includes, for example, increased dosages of the antibody or antigen-binding fragment thereof, increased frequency of dosing, or both. An acute phase of treatment may include, for example, intravenously or subcutaneously administering the antibody or antigen-binding fragment thereof at a dosage of from about 5 mg/kg to about 10 mg/kg (e.g., about 5 mg/kg, 6 mg/kg, 7 mg/kg, 8 mg/kg, 9 mg/kg, or 10 mg/kg), for example, once every two weeks, once every week, or twice every week, or more. The subject can then be monitored for a reduction (e.g., by at least 10%, 20%, 30%, 40%, 50%, 60%, 70%, 80%, 90%, or 100%) of one or more symptoms associated with RA. The inhibition or reduction of a symptom may be measured with respect to a control subject (e.g., a healthy subject or a different subject with RA that does not undergo a treatment regimen as described herein) or relative to the subject prior to the acute phase of treatment. The reduction may include a reduction in frequency and/or intensity of symptoms, e.g., as measured by an assessment as described herein. A reduction in frequency and/or amount of the antibody or antigen-biding fragment thereof may be determined based on an efficacy metric as described herein (e.g., DAS28, DAS28 CRP, or ACR 20/50/70 assessment). For example, if the subject achieves a DAS28 score of <3.2, the subject may be determined to require a reduced frequency or dosage of the antibody or antigen-biding fragment thereof.

After the acute phase of treatment, e.g., that results in reduction of one or more symptoms of RA or a reduced DAS28 score (e.g., of less than 2.6 or less than 3.2), the subject may then transition to a chronic dosing regimen that includes, for example, a reduced dosage of the antibody or antigen-binding fragment thereof, reduced frequency of dosing, or both, relative to the acute phase of treatment. For example, a chronic phase of treatment may include, for example, intravenously or subcutaneously administering the antibody or antigen-binding fragment thereof at a dosage of from about 1 mg/kg to about 5 mg/kg (e.g., about 1 mg/kg, 1.5 mg/kg, 2 mg/kg, 2.5 mg/kg, 3 mg/kg, 3.5 mg/kg, 4 mg/kg, 4.5 mg/kg, or 5 mg/kg), for example, once every four weeks, once every two weeks, or once every week.. Such a chronic dosing regimen may provide for a reduced overall dosage administered to the subject while still maintaining therapeutic efficacy.

The subject may transition from acute dosing to chronic dosing (e.g., when a reduction in one or more RA symptoms is observed) or from chronic dosing to acute dosing (e.g., when a flare-up of one or more RA symptoms is observed) one or more times during treatment with the anti-CD40 antibody or antigen-binding fragment thereof, e.g., as needed based on the observed RA symptom(s) in the subject.

In certain embodiments, the subject is a responder to the treatment with the anti-CD40 antibody or antigen-binding fragment thereof and is identified as having a statistically significant improvement in disease activity, wherein the disease activity is determined by one or more criteria selected from the group consisting of ACR20, ACR50, ACR70, DAS28, DAS28-CRP, Physician's Global Assessment (PhGA) of disease activity, Health Assessment Questionnaire-Disability Index (HAQ-DI), Short Form Health survey (SF-36) in the physical health composite score (PCS) and mental health composite score (MCS), SF-36 total composite score (TCS), Clinical Disease Activity Index (CDAI), SDAI (derived from CDAI plus DAS28-CRP, and a pain Visual Analog Scale (VAS). In particular embodiments, a subject achieves a significant improvement in ACR20 response for KPL-404 vs. placebo by week 12, by week 24, or by week 52 of the treatment. In other embodiments, a subject achieves a significant improvement in ACR50 response for KPL-404 vs. placebo by week 12, by week 24, or by week 52 of the treatment. In certain embodiments, a subject achieves a significant improvement in DAS28-CRP response for KPL-404 vs. placebo by week 12, by week 24, or by week 52 of the treatment.

### Combination Therapy

The pharmaceutical compositions administered in a method as described herein can be administered alone or in combination with one or more other therapeutic agents (e.g., a second therapeutic agent). In some embodiments, the pharmaceutical composition including the anti-CD40 antibody or antigen-binding fragment thereof can further include a second therapeutic agent, e.g., either conjugated or unconjugated to the antibody or its fragment. In one embodiment, the second agent is another monoclonal or polyclonal antibody or antigen-binding fragment thereof. In another embodiment, the second agent is an immunosuppressant. In one embodiment, the second agent is a cytotoxic or cytostatic agent. In another embodiment, the second agent may target a receptor or receptor complex other than CD40 on the surface of activated lymphocytes, dendritic cells or CD40-expressing cancer cells.

Such combination therapy can have an additive or synergistic effect on condition parameters (e.g., severity of a symptom, the number of symptoms, or frequency of relapse).

The pharmaceutical composition may be administered concurrently with the second therapeutic agent. In another specific embodiment, the second therapeutic agent is administered prior to or subsequent to administration of the pharmaceutical composition containing the anti-CD40 antibody or antigen-binding fragment thereof.

The pharmaceutical composition may be formulated with or administered in combination with a disease-modifying antirheumatic drug (DMARD), e.g., for the treatment of rheumatoid arthritis. For example, the pharmaceutical composition may be administered with one or more of abatacept, adalimumab, anakinra, apremilast, azathioprine, baricitinib, certolizumab pegol, chloroquine, ciclosporin (Cyclosporin A), D-penicillamine, etanercept, filgotinib, golimumab, gold salts (e.g., sodium aurothiomalate or auranofin), hydroxychloroquine, infliximab, leflunomide, methotrexate, minocycline, rituximab, sarilumab, secukinumab, sulfasalazine, tocilizumab, tofacitinib, and ustekinumab.

In some embodiments, the DMARD is a conventional synthetic DMARD.

The pharmaceutical compositions containing the antibodies or antigen-binding fragments described herein can be formulated or administered in combination with an immunosuppressant. Examples of immunosuppressants include, but are not limited to, calcineurin inhibitors (e.g., a cyclosporin, such as, e.g., cyclosporin A (SANDIMMUNE^{®}) andcyclosporine G tacrolimus (PROGRAF^{®}, PROTOPIC^{®})), an mTor inhibitor (e.g., sirolimus (RAPAMUNE^{®}, NEORAL^{®}), temsirolimus (TORISEL^{®}), zotarolimus, and everolimus (CERTICAN^{®})), fingolimod (GILENYA^{™}), myriocin, alemtuzumab (CAMPATH^{®}, MABCAMPATH^{®}, CAMPATH-1H^{®}), rituximab (RITUXAN^{®}, MABTHERA^{®}), an anti-CD4 monoclonal antibody (e.g., HuMax-CD4), an anti-LFA1 monoclonal antibody (e.g., CD11a), an anti-LFA3 monoclonal antibody, an anti-CD45 antibody (e.g., an anti-CD45RB antibody), an anti-CD19 antibody (see, e.g., U.S. Patent Publication 2006/0280738), monabatacept (Orencia^{®}), belatacept, indolyl-ASC (32-indole ether derivatives of tacrolimus and ascomycin), azathioprine (AZASAN^{®}, IMURAN^{®}), lymphocyte immune globulin and anti-thymocyte globulin [equine] (ATGAM^{®}), mycophenolate mofetil (CELLCEPT^{®}), mycophenolate sodium (MYFORTIC^{®}), daclizumab (ZENAPAX^{®}), basiliximab (SIMULECT^{®}), cyclophosphamide (ENDOXAN^{®}, CYTOXAN^{®}, NEOSAR^{™}, PROCYTOX^{™}, REVIMMUNE^{™}), prednisone, prednisolone, leflunomide (ARAVA^{®}), FK778, FK779, 15-deoxyspergualin (DSG), busulfan (MYLERAN^{®}, BUSULFEX^{®}), fludarabine (FLUDARA^{®}), methotrexate (RHEUMATREX^{®}, TREXALL^{®}), etanercept (ENBREL^{®}), adalimumab (HUMIRA^{®}), 6-mercaptopurine (PURINETHOL^{®}), 15-deoxyspergualin (Gusperimus), LF15-0195, bredinin, brequinar, and muromonab-CD3 (ORTHOCLONE^{®}).

The pharmaceutical compositions containing the antibodies or antigen-binding fragments described herein can be formulated or administered in combination with a Janus Kinase (JAK) inhibitor. The JAK inhibitor may be, for example, ruxolitinib, tofacitinib, oclacitinib, baricitinib, peficitinib, fedratinib, upadacitinib, filgotinib, or delgocitinib,

The pharmaceutical compositions containing the antibodies or antigen-binding fragments described herein can be formulated or administered in combination with a corticosteroid (e.g., prednisone).

The antibody or antigen-binding fragment thereof and the second therapeutic agent (e.g., an antirheumatic drug) may be administered within one month of each other or within one week of each other.

### Examples

The following examples of specific aspects for carrying out the present disclosure are offered for illustrative purposes only and are not intended to limit the scope of the present disclosure in any way.

### Example 1

### Identification of Chronic Dosing Regimens in Patients with Rheumatoid Arthritis by PK Analysis and Modeling of KPL-404.

In a first-in-human Phase 1 single ascending dose study, 52 healthy volunteers received single doses of KPL-404 administered either subcutaneously (SC) or intravenously (IV) with no dose-limiting safety findings, infectious episodes, or toxicities. The study demonstrated that with 10 mg/kg IV, full receptor occupancy (RO) was observed through day 71, and there was complete suppression of T-cell dependent antibody response (TDAR) to keyhole limpet hemocyanin challenge on day 1 and re-challenge on day 29 through day 57. With 5 mg/kg SC, full RO was observed through day 43, and there was complete suppression of TDAR through at least day 29. Complete suppression of anti-drug antibodies (ADA) to KPL-404, an independent indicator of target engagement, was also observed while serum concentrations of KPL-404 were above approximately 0.1 to 0.2 µg/mL and continued for at least 50 days and 57 days after 5 mg/kg SC and 10 mg/kg IV administration, respectively.
**Objective:** Using Phase 1 and nonclinical data, identify chronic dosing regimens that yield PK in the sub-therapeutic, therapeutic, and supra-therapeutic ranges to be utilized in a Multiple Ascending Dose Phase 2 Study.

### Methods

A PK model was used to simulate multiple dosing scenarios, including: 2.5, 5, and 10 mg/kg SC qwk, q2wk, and q4wk, as well as 10 mg/kg IV q4wk. The PK model was used to identify therapeutic dosing schedules by generating 1000 virtual subjects using parameter estimates with between-subject variability included. Following SC administration, all subjects were predicted to achieve complete ADA suppression for the full dosing interval at/above 2.5 mg/kg SC q2wk.

### Results

At 2 mg/kg SC q2wk (starting dose level), simulated steady-state 8-week data predicted PK in a sub-therapeutic range for most subjects and an approximately 31- and 18-fold safety margin relative to preclinical NOAEL dose. At 5 mg/kg SC q2wk, 100% of patients were predicted to be in a therapeutic range, indicating a potential practical efficacious dose level. At 10 mg/kg SC q2wk, 100% of patients were predicted to be in the supratherapeutic range.

Following SC administration, all subjects were predicted to achieve complete ADA suppression for the full dosing interval at/above 2.5 mg/kg SC q2wk.

At 2 mg/kg SC q2wk (starting dose level), simulated steady-state 8-week data predicted PK in a sub-therapeutic range for most subjects and an approximately 31- and 18-fold safety margin relative to preclinical NOAEL dose.

At 5 mg/kg SC q2wk, 100% of patients were predicted to be in a therapeutic range, indicating a potential practical efficacious dose level.

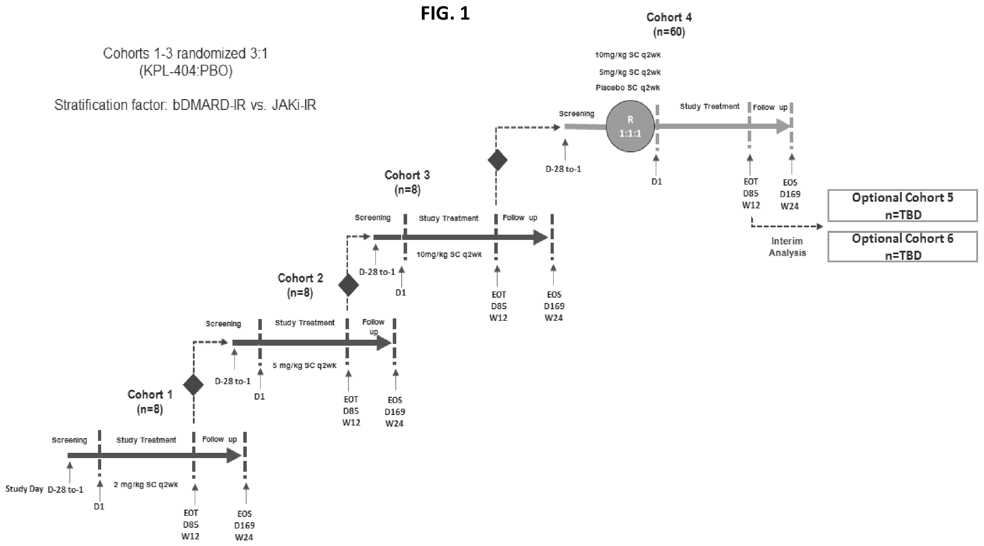

At 10 mg/kg SC q2wk, 100% of patients were predicted to be in the supratherapeutic range. These results support a Multiple Ascending Dose (MAD) Phase 2 study design, with PK lead-in comprised of 3 Cohorts at 2, 5, or 10 mg/kg SC q2wk (each randomized 6:2) and Proof-of-Concept phase (Cohort 4) comprised of 48-60 subjects randomized 1:1:1 to 10 mg/kg, 5 mg/kg, and placebo SC q2wk (FIG.1). The study was designed to evaluate efficacy (Disease Activity of 28 joints using C-reactive protein (DAS28-CRP)), safety, PK, and pharmacodynamics (PD) of escalating doses levels of KPL-404 compared with placebo in patients with moderate to severe RA (bDMARD-IR or JAKi-IR). The study also allows the flexibility of optional cohorts including additional dosing regimens and/or subpopulations identified based on clinical response and biomarkers.

### Conclusion

Inhibition of the CD40-CD154 co-stimulatory interaction can be used to manage a spectrum of rheumatoid arthritis (RA). KPL-404 demonstrated prolonged absorption/excretion capable of suppressing TDAR for extended periods allowing for use of extended dosing intervals irrespective of IV or SC dosing. These analyses demonstrate the efficacy and safety of KPL-404 in treating RA, in particular in a chronic dosing regimen.

### Example 2

### Study to Assess the Safety, Pharmacokinetics (PK), and Efficacy of KPL-404 in Subjects with Rheumatoid Arthritis (RA) With Inadequate Response or Intolerance to at Least One Biologic Disease-modifying Anti Rheumatic Drug (bDMARD) or a Janus Kinase Inhibitor (JAKi)

Rheumatoid arthritis (RA) is a chronic systemic inflammatory disease of unknown etiology. The hallmark of RA is an inflammatory process manifested by persistent symmetric polyarthritis of synovial joints, which can ultimately lead to bone erosions, deformity, and disability. Left untreated, or inadequately treated, progressive functional impairment with increasing disability occurs, leading to decreased ability to perform activities of daily living resulting in a reduction in quality of life. The prevalence of RA in the general population is approximately 1% and increases with age in both sexes, with women being more prone to developing RA than men. Standard-of-care treatment includes early therapy with disease-modifying anti-rheumatic drug (DMARDs), including conventional synthetic DMARDs (csDMARDs) (e.g., methotrexate, sulfasalazine, hydroxychloroquine, and leflunomide). In the last 2 decades, targeted biological DMARDs (bDMARDs) have revolutionized the treatment paradigm for RA, in which therapy is optimized until clinical remission or at minimum low disease activity is achieved. The bDMARDs have greatly improved the clinical, functional, and radiographic outcomes for subjects with RA. Despite a wide therapeutic armamentarium of bDMARDs, a blockade of any single cytokine or cellular subset has not shown control of disease in all RA subjects. In randomized controlled studies and clinical practice, approximately 30% to 40% of treated subjects show inadequate response to bDMARDs, fail to maintain good response over time, or experience adverse events (AEs) leading to treatment withdrawal. These subjects with inadequate response or intolerance to one or more bDMARDs, in many of whom multiple csDMARDs have previously failed, have few treatment options, and treatment of the disease in this population remains a crucial unmet need in the management of RA.

The safety, pharmacokinetics (PK), and pharmacodynamics (PD) of KPL-404 have been assessed in a single ascending dose study in healthy subjects. The KPL-404 will be administered initially as doses of up to 10 mg/kg administered subcutaneously (SC) on alternate weeks (q2wk). Once safety and tolerability have been established in Cohorts 1, 2, and 3, Cohort 4 is designed to assess the efficacy of KPL-404 using expanded numbers of treated subjects (Cohorts 1-3 have approximately 24 subjects, and Cohort 4 has up to 60 subjects; additional Cohorts may be tested). Efficacy will be assessed using, as a primary endpoint, change from baseline in disease activity score of 28 joints using C-reactive protein (DAS28-CRP) at 12 weeks. Additional cohorts may be initiated at a dose and regimen that will not exceed the overall exposure associated with the 10 mg/kg q2wk dosing regimen.

### Dosing

The Cohort 1 starting dose was selected based on the following factors.

The safety profile of the single ascending dose study in healthy subjects: single doses up to 10 mg/kg IV and 5 mg/kg SC were assessed. There were no deaths or related serious adverse events ([SAEs] the only serious event being patellar fracture related to a fall). There were no dose-limiting toxicities observed.

Analysis of the pharmacokinetic data from previous trials with KPL-404 indicate that the Cₘₐₓ and AUC_{0-∞} associated with the 3.0 mg/kg IV dose were approximately 3.1-fold and 4.7-fold lower than the 10 mg/kg IV dose, respectively. The expected Cₘₐₓ and the end of dosing interval (AUC₀₋ₜ) associated with the starting dose of ≤ 2.5 mg/kg q2wk are approximately 7- and 8-fold lower than the 10 mg/kg single IV dosing regimen in the Phase 1 study. Additionally, the starting dose of ≤ 2.0 mg/kg SC is expected to show lower variability compared to lower doses (≤ 1 mg/kg) because the influence of target-mediated drug disposition (TMDD) on the PK profile is expected to be minimal.

KPL-404 was evaluated in 8-week and 26-week repeated-dose toxicity studies in cynomolgus monkeys. The only adverse findings in the studies were an early termination on Day 143 of one female at 97 mg/kg SC after exhibiting clinical signs of poor health for several days, and another female administered 97 mg/kg SC that exhibited bilateral uveitis associated with a small blood clot in the anterior chamber of the eye. The no-observed-adverse-effect level (NOAEL) is 12-fold higher than the starting dose in this Phase 2 study, and the systemic exposure associated with the NOAEL by the SC route was approximately 10.6-fold higher. This provides an adequate safety margin between the proposed dose range for this study relative to the nonclinical safety findings.

### Benefit/Risk Assessment

The results of nonclinical studies and the completed Phase 1 study show that KPL-404 dosing of up to 10 mg/kg biweekly is safe. Given the population of subjects who have failed to achieve an adequate response to other RA treatments, PK, and safety data from the Phase 1 first-in-human study, and nonclinical data, KPL-404 has the potential to improve outcomes in subjects with RA (such as previously non-responsive subjects), with low risk of significant adverse events.

**TABLE 2: STUDY OBJECTIVES AND ENDPOINTS**

| **Primary Objectives:** | **Primary Endpoints:** | | |
|---|---|---|---|
| Cohorts 1, 2, and 3: To evaluate the dose-response relationship as measured by safety, tolerability, and pharmacokinetics of multiple SC doses of KPL-404 versus placebo. | Cohorts 1, 2, and 3: | | |
| | | • Incidence of treatment-emergent adverse events (TEAEs) | |
| | | • Maximum serum concentration (Cₘₐₓ) | |
| | | • AUC₀₋ₜ | |
| Cohort 4 in conjunction with data from Cohorts 1 (placebo only), 2, and 3: To evaluate the efficacy of KPL-404 versus placebo for the treatment of RA. | | • Cohort 4 in conjunction with data from Cohorts 1 (placebo only), 2, and 3: Change from baseline in DAS28-CRP at Week 12 | |

| **Secondary Objectives:** | | **Secondary Endpoints:** | |
|---|---|---|---|
| **Primary Objectives:** | **Primary Endpoints:** | | |
| Cohorts 1, 2, and 3: To contribute to the evaluation of the efficacy of multiple SC doses of KPL-404 versus placebo for the treatment of RA. | | • Cohorts 1, 2, and 3: Change from baseline in DAS28-CRP at Week 12 | |
| Cohort 4 only: To evaluate the efficacy of KPL-404 versus placebo for the treatment of RA. | | • Cohort 4 only: Change from baseline in DAS28-CRP at Week 12 | |
| Cohort 4: To evaluate the safety, tolerability, and PK of KPL-404 versus placebo. | Cohort 4: | | |
| | | • Incidence of TEAEs | |
| | | • Cmax | |
| | | • AUC₀₋ₜ | |

| **Other Objective:** | **Other Endpoints (All Cohorts):** | | |
|---|---|---|---|
| To evaluate the effect of KPL-404 on disease activity and biomarkers versus placebo as appropriate. | | • Vital signs and ECGs over time | |
| | | • Changes from baseline in clinical laboratory test results | |
| | | • Proportion of subjects achieving ACR20 responses at Week 12 | |
| | | • Change from baseline in DAS28-CRP over time | |
| | | • Changes from baseline in clinical disease activity index (CDAI) and simplified disease activity index (SDAI) over time | |
| | | • Proportion of subjects achieving low disease activity over time as defined by the following 3 independent endpoints: | |
| | | | ∘ DAS28-CRP (≤ 3.2) |
| | | | ∘ CDAI (≤ 10.0) |
| | | | ∘ SDAI (≤ 11.0) |
| | | • Proportion of subjects achieving complete response (CR) over time as defined by the following 3 independent endpoints: | |
| | | | ∘ DAS28-CRP (< 2.6) |
| | | | ∘ CDAI (≤ 2.8) |
| | | | ∘ SDAI (≤ 3.3) |
| | | • American College of Rheumatology (ACR) response rates over time: | |
| | | | ∘ ACR20 |
| | | | ∘ ACR50 |
| | | | ∘ ACR70 |
| | | • Change from baseline in individual components of ACR and DAS28-CRP response over time | |
| | | • Change from baseline in Subject's Assessment of Pain (visual analog scale; VAS) over time | |
| | | • Change from baseline in Short Form-36 (SF-36) Physical Component Score over time | |
| | | • Change from baseline in autoantibodies (rheumatoid factor [RF], anti-citrullinated protein antibody [ACPA]) over time | |
| | | • Change from baseline in circulating and synovial tissue biomarkers, including pharmacodynamic biomarkers over time as appropriate | |
| | | • Time from randomization to start of rescue medications | |
| | | • Time from last dose for subjects who have completed treatment to start of rescue medications | |

| **Primary Objectives:** | **Primary Endpoints:** | | |
|---|---|---|---|
| | | • Proportions of subjects needing rescue medication from randomization | |
| | | • Proportions of subjects completing treatment that need rescue medication after last dose | |
| All Cohorts: To evaluate the immunogenicity of KPL-404 versus placebo | | • Anti-drug antibody (ADA) titers | |

### INVESTIGATIONAL PLAN

### Overall Study Design

FIG. 1 displays the overall scheme for the study. A detailed outline of procedures and activities is provided in Table 3.

**Table 3. Schedule of Activities**

| | **Study Period** | **Screening Period** | **Treatment Period** | | | | | | | | **Safety Follow up Period** | | | |
|---|---|---|---|---|---|---|---|---|---|---|---|---|---|---|
| | **Week** | **Screening** | **Baseline** | **Wk 1** | **Wk 2** | **Wk 4** | **Wk 6** | **Wk 8** | **Wk 10** | **Wk 12/ EOT^{a}** | **Wk 14** | **Wk 16** | **Wk 20** | **Wk 24/EOS** |
| | **Day** | **-28 to 1** | **1** | **8** | **15** | **29** | **43** | **57** | **71** | **85** | **99** | **113** | **141** | **169** |
| | **Visit Window** | | | **± 2 days** | **± 2 days** | **± 2 days** | **± 2 days** | **± 2 days** | **± 2 days** | **± 2 days** | **± 2 days** | **± 2 days** | **± 2 days** | **± 4 days** |
| Informed consent | | X | | | | | | | | | | | | |
| Demographics | | X | | | | | | | | | | | | |
| Eligibility criteria | | X | X | | | | | | | | | | | |
| Medical/surgical history^{b} | | X | X | | | | | | | | | | | |
| Randomization | | | X | | | | | | | | | | | |
| **Safety assessments** | | | | | | | | | | | | | | |
| | Adverse events^{c} | X | X | X | X | X | X | X | X | X | X | X | X | X |
| | Chest x-ray^{d} | X | | | | | | | | | | | | |
| | 12-lead ECG | X^{d} | | | | | | | | X | | | | |
| | Physical examination^{e} | X | X | | | | | | | X | | | | |
| | Vital signs^{f} | X | X | | X | X | | X | | X | | | | |
| | Height (screening only) and weight | X | X | | | | | | | X | | | | |
| | Prior/concomitant medications and therapies | X | X | X | X | X | X | X | X | X | X | X | X | X |
| **RA assessments** | | | | | | | | | | | | | | |
| | TJC68/SJC66 | X | X | | X | X | | X | | X | X | X | X | X |
| | Physician's Global Assessment (VAS) | | X | | X | X | | X | | X | X | X | X | X |
| | Subject-reported outcomes^{g} | | | | | | | | | | | | | |
| | Subject Global Assessment (VAS) | | X | | X | X | | X | | X | X | X | X | X |
| | Pain (VAS) | | X | | X | X | | X | | X | X | X | X | X |
| | HAQ-DI | | X | | X | X | | X | | X | X | X | X | X |
| | SF-36 PCS | | X | | X | X | | X | | X | X | X | X | X |
| **Blood collection:** | | | | | | | | | | | | | | |
| | QuantiFERON test | X | | | | | | | | | | | | |
| | HBV/HCV | X | | | | | | | | | | | | |
| | HIV (local laboratory)^{h} | X | | | | | | | | | | | | |
| | Serum β-hCG for WOCBPⁱ | X | | | | | | | | | | | | X |
| | Central hs-CRP | X | X | | X | X | | X | | X | X | X | X | X |
| | RF, ACPA | X | X | | X | X | | X | | X | X | X | X | X |
| | Blood chemistry^{j} | X | X | | X | X | | X | | X | | | | |
| | Hematology (CBC) with differential | X | X | | X | X | | X | | X | | | | |
| | Coagulation panel | X | X | | X | X | | X | | X | | | | |
| | Circulating biomarkers^{k} | | X | | X | X | | X | | X | X | X | X | X |
| | Circulating transcriptomic biomarkers^{k} | | X | | | | | | | X | | | | |
| | Synovial tissue biopsy^{I} | | X | | | | | | | | | | | |
| | Pharmacokinetic samples (serum) | | X | X | X | X | X | X | X | X | X | X | X | X |
| ADA samples (serum) | | | X | | | X | X | X | X | X | X | X | X | X |
| **Urine collection:** | | | | | | | | | | | | | | |
| | Urinalysis^{m} | X | X | | X | X | | X | | X | | | | |
| | Urine β-hCG for WOCBP (local)ⁿ | | X | | | X | | X | | X | | X | X | X |
| **IP administration** | | | X | | X | X | X | X | X | | | | | |

| | | | | | | | | | | | | | | |
|---|---|---|---|---|---|---|---|---|---|---|---|---|---|---|
| AE = adverse event; ACPA = anti-citrullinated protein antibody; ADA = anti-drug antibody; β-hCG = beta-human chorionic gonadotropin; ECG = electrocardiogram; EOS = end of study; EOT = end of treatment; HAQ-DI = Health Assessment Questionnaire Disability Index; HBV = hepatitis B virus; HCV = hepatitis C virus; HIV = human immunodeficiency virus; hs-CRP = high-sensitivity C-reactive protein; IP = investigational product; PCS = Physical Component Score; RA = rheumatoid arthritis; RF = rheumatoid factor; SAE = serious adverse event; SF-36 = 36-Item Short Form Health Survey; SJC = swollen joint count; TJC = tender joint count; Wk = week; WOCBP = women of childbearing potential; VAS = visual analog scale. a If a subject prematurely discontinues prior to the completion of the treatment period (Week 12), the procedures for the EOT visit should be conducted within approximately 2 weeks after discontinuation, and the subject should be encouraged to complete the remaining visits, up to the Week 24 visit. b Prior medication will be recorded up to 60 days prior to screening, prior medication for RA will be recorded up to 1 year prior to screening, and COVID-19 vaccine regimen at least 3 weeks before the first dose of IP. Medical history to include drug or alcohol abuse within the last 6 months, RA history, cancer within the last 5 years from screening, and surgical history. c During the screening period, only SAEs and protocol-related nonserious AEs will be recorded. d The chest x-ray and ECG will not be required if a subject had a previously documented normal chest x-ray/ECG within 120 days of screening. e Complete physical examination (minus genitourinary/pelvic examination) at screening and EOS; abbreviated examination at baseline and other time points if deemed indicated by investigator. f Blood pressure, pulse rate, and body temperature should be measured before blood draws are performed. g Subject-reported outcomes performed prior to other procedures. h Subjects who have not had an HIV test within 8 weeks of screening will be tested. Subjects with tests results indicating positive HIV infection will not be eligible for study participation. i If serum pregnancy test result is borderline, a repeat test is necessary to confirm eligibility. If still borderline ≥ 3 days later, this will be considered documentation of continued lack of a positive result, and the subject can be enrolled into the study. j Minimum 8-hour fast. If a subject is unable to fast when necessary, the nonfasting status will be recorded. k Samples will be collected as required for circulating biomarker collection. Refer to the Laboratory Manual for specific collection tubes, sample volume, sample types, and collection processes. I A synovial tissue biopsy is required only for subjects consenting to the biopsy substudy at specific sites participating in biopsy substudy. The investigator will have the option to collect second biopsy from Week 4 through the Week 8 visit. m A urine dipstick macroscopic urinalysis will be completed by the central laboratory at all required visits. n If urine pregnancy test is positive, withhold dosing and perform a serum pregnancy test. Pregnant subjects must permanently discontinue IP. | | | | | | | | | | | | | | |

KPL-404 therapy will be explored in a 28-week (up to 4-week screening period, 12-week treatment period, and 12-week safety follow-up period), multicenter, randomized, double-blind, placebo-controlled, multiple dose study with PK lead-in designed to assess the safety, PK, efficacy, and PD of KPL-404 in subjects with moderate to severe, active RA who have inadequate response to or are intolerant to a JAKi or at least one bDMARD. Subjects who have been treated with both bDMARD and JAKi are excluded from the study. The objectives of the study are to evaluate safety, efficacy, and PD compared with placebo across the estimated therapeutic range and to characterize PK across varying dose levels of KPL-404.

Subjects will enter the screening period (Day -28 to -1), before returning for a randomization visit and administration of the first dose (Day 1). Subjects can be rescreened only once, at the discretion of the investigator and with approval from the medical monitor. Abnormal laboratory values may in certain cases be repeated with the approval of the medical monitor without the need for rescreening. If a subject is rescreened, a new subject number will be provided, and laboratory assessments can be repeated once where appropriate.

All subjects will receive an SC injection of the investigational product (IP) on Day 1 at the study site. In the first 3 cohorts, subjects will be randomized in a 3:1 ratio to escalating doses as follows:
- Cohort 1: 2 mg/kg KPL-404 or placebo every 2 weeks (q2wk) (n = 8)
- Cohort 2: 5 mg/kg KPL-404 or placebo q2wk (n = 8)
- Cohort 3: 10 mg/kg KPL-404 q2wk or placebo (n = 8)

Cohorts 1 to 3 will start sequentially and each will be assessed in an unblinded fashion for safety and tolerability data after all subjects complete through Week 12 and PK through Week 8 (anticipated study state) and applicable data are available.

Following review of the safety, tolerability, and PK data as described above, subjects will be enrolled in Cohort 4. Cohort 4 subjects will be randomized 1:1:1 to receive:
- 10 mg/kg KPL-404 q2wk (n = 16-20)
- 5 mg/kg KPL-404 q2wk (n = 16-20)
- Placebo q2wk (n = 16-20)

Subjects will be observed for at least 60 minutes following the first administration and 30 minutes after subsequent administrations. If no safety concerns are identified during that time, subjects will be discharged from the study site after all study procedures have been completed. All subjects will return to the study site at all treatment and safety follow-up visits as per the Schedule of Activities (Table 3) for assessments of RA parameters as well as evaluations of safety, tolerability, PK, biomarkers, and ADAs.

An optional synovial biopsy substudy may be conducted at selected study sites. Participation in the substudy will be optional. Synovial biopsies from subjects participating in the substudy will be collected at time points noted in the schedule of assessments. Biopsy collection procedures and processing will be outlined in a separate manual.

If a subject discontinues prematurely (i.e., before the end-of-treatment visit), the subject should be encouraged to complete the procedures for the end-of-study visit (Week 12) (within approximately 2 weeks after study drug discontinuation) as well as visits thereafter through the Week 24 visit.

At the end of the 12-week treatment period, all subjects will be followed up for safety for 30 days during which adverse event (AE) data will be collected. In order to investigate the longevity of any treatment effect observed, all subjects will then be followed for a further 8 weeks (for a total of 12 weeks off treatment observation) during which time data will be collected for PK assessment as well as disease status and concomitant medications.

### Number of Subjects

The study plans to enroll approximately 24 subjects in Cohorts 1 to 3 and up to 60 subjects in Cohort 4, across 100 study sites. Additional Cohorts may be assigned and studied. All subjects will be assigned to randomized study drug.

A subject may be discontinued from treatment due to any Grade 3 (severe) or any Grade 4 (life-threatening) TEAE regardless of causality.

For the study, enrollment in a cohort will be paused for review under any of the circumstances below:
- Two subjects develop the same Grade 3 TEAE
- One subject has a Grade 4 (life-threatening) or Grade 5 (fatal) TEAE

If appropriate, after review of the TEAE, the study will resume.

### SELECTION AND WITHDRAWAL OF SUBJECTS

### Subject Inclusion Criteria

For inclusion in the study, subjects must fulfill all of the following criteria:
1. Adult male or female, age 18 to 80 years of age (inclusive).
2. Body weight ≥ 40 to ≤ 100 kg for all cohorts.
3. Diagnosis of RA for ≥ 3 months fulfilling the 2010 American College of Rheumatology (ACR)/European Union League Against Rheumatism (EULAR) classification criteria for RA and that is categorized as ACR RA functional Class 1-3.
4. Treated with a biological disease-modifying anti-rheumatic drug (bDMARDs) OR Janus kinase inhibitor (JAKi) therapy for RA for ≥ 3 months and had inadequate response or had to discontinue bDMARD OR JAKi therapy due to intolerance or toxicity, regardless of treatment duration.
5. Currently receiving csDMARD therapy ≥ 3 months and on a stable dose for ≥ 4 weeks before the first dose of investigational product.
   a. The following csDMARDs are allowed: oral or parenteral methotrexate ([MTX]; 7.5 to 25 mg/week), sulfasalazine (≤ 3000 mg/day), hydroxychloroquine (≤ 400 mg/day), chloroquine (≤ 250 mg/day), and leflunomide (≤ 20 mg/day).
   b. A combination of up to 2 background csDMARDs is allowed, except the combination of MTX and leflunomide.
6. Meets all of the following disease activity criteria:
   a. Six or more swollen joints (based on 66 joint counts) and ≥ 6 tender joints (based on 68 joint counts) at screening and baseline visits;
   b. Level of high-sensitivity C-reactive protein ≥ 5 mg/L (by central laboratory);
   c. Seropositivity for serum RF and/or ACPA at screening, according to the central laboratory's definition of positivity.
7. Must have discontinued all bDMARDs or JAKi prior to the first dose of KPL-404. The washout period for bDMARDs or JAKi prior to the first dose of KPL-404 is specified below. For bDMARDs or JAKi not listed below washout should be at least 5 times the mean elimination half-life of a drug:
   a. ≥ 4 weeks for etanercept;
   b. ≥ 8 weeks for adalimumab, infliximab, certolizumab, golimumab, abatacept, tocilizumab, and sarilumab;
   c. ≥ 1 year for rituximab;
   d. ≥ 2 weeks for JAKi (either investigational or commercially available treatment).
8. Must have discontinued all high-potency opiates at least 1 week for prior to the first dose of KPL-404.
9. If female, must be either postmenopausal, permanently surgically sterile, or, for women of childbearing potential, must practice at least one highly effective method of contraception that is effective from study Day 1 through at least 30 days after the EOS visit (additional local requirements may apply). Sexually active female subjects must be:
   - Nonpregnant, nonlactating, and having agreed to use a highly effective method of contraception from the screening visit until 30 days after EOS visit.
      ∘ Note: highly effective methods of contraception include:
         • hormonal contraceptives associated with inhibition of ovulation (stable dose for at least 4 weeks prior to first dose)
         ▪ intrauterine device
         ▪ intrauterine system
         ▪ bilateral tubal occlusion
         ▪ vasectomized male partner
         ▪ sexual abstinence
10. Sexually active male subjects must have documented vasectomy or must agree to use a highly effective method of contraception as defined above with their partners of childbearing potential from first dose until 30 days after EOS visit.
11. Male subjects must agree to refrain from donating sperm from first dose until 30 days after the last study drug administration. Female subjects must agree to refrain from donating eggs from first dose until 30 days after EOS visit.
12. Female of childbearing potential must have a negative serum β-human chorionic gonadotropin (β-hCG) test at the screening visit and negative urine pregnancy test on Day 1.

### Subject Exclusion Criteria

The subject may not enter the study if ANY of the following apply:
1. Prior exposure to any other anti-CD40/CD40L agent.
2. Prior treatments with both a bDMARD and a JAKi.
3. Injectable corticosteroids (including intra-articular) or treatment with > 10 mg/day dose oral prednisone or equivalent within 8 weeks prior to randomization. (Note: Concomitant treatment with nonsteroidal anti-inflammatory drugs, acetaminophen, oral corticosteroids [equivalent to prednisone ≤ 10 mg/day], or inhaled corticosteroids at a stable dose ≥ 4 weeks prior to baseline for stable medical conditions is allowed and should be kept at a stable dose throughout the study.)
4. Has received any investigational product within 30 days or 5 half-lives if the half-life is known of the investigational product (whichever is longer) before the first dose investigational product.
5. Concurrent enrollment in another clinical study, with the exception of observational studies.
6. Major surgery within 8 weeks prior to screening or planned major surgery within 6 months after baseline.
7. Transplanted organs (except corneal transplant performed more than 3 months prior to baseline).
8. Receipt of live (attenuated) vaccine within the 4 weeks before baseline or expected need of live vaccination during study participation including 4 weeks after the last dose of KPL-404.
9. History of any arthritis with onset prior to age 16 years or current diagnosis of inflammatory joint disease other than RA (including but not limited to gout, systemic lupus erythematosus, psoriatic arthritis, axial spondyloarthritis including ankylosing spondylitis and nonradiographic axial spondyloarthritis, reactive arthritis, overlap connective tissue diseases, scleroderma, polymyositis, dermatomyositis, fibromyalgia [currently with active symptoms]). Current diagnosis of secondary Sjogren's syndrome is permitted.
10. Clinically significant active infection including signs/symptoms suggestive of infection, any significant recurrent or chronic infection (including positive hepatitis C virus antibody), or any episode of infection requiring hospitalization or treatment with IV antibiotics within 12 weeks before screening, or treatment with oral anti-infectives within 14 days prior to the first dose of KPL-404. Subjects with any opportunistic infection within 6 months before screening will be excluded from the study.
11. Subjects at a high risk of infection (e.g., history of hereditary or acquired immune deficiency disorder), a history of an infected joint prosthesis at any time with that prosthesis still in situ, leg ulcers, indwelling urinary catheter, or persistent or recurrent chest infections.
12. Subjects testing positive for human immunodeficiency virus infection.
13. Subjects with chronic active hepatitis B infection as defined below will be excluded from the study:
   a. Hepatitis B surface antigen positive.
   b. Hepatitis B anti-core antibody positive but anti-surface antibody negative.
14. Positive (or 2 indeterminate) QuantiFERON^{®} test results (when possible, test should be performed at least 4 weeks after receiving an mRNA COVID-19 vaccine).
15. History of thromboembolic event, a significant risk of future thromboembolic events (defined as a definitive diagnosis of thrombophilia OR an unstable condition associated with an increased incidence of thrombosis, such as atrial fibrillation or presence of anti-phospholipid antibodies). All history of thrombosis should be approved by the medical monitor.
16. Laboratory values meeting the following criteria within the screening period (prior to randomization) of KPL-404:
   a. Serum aspartate aminotransferase > 3 × upper limit of normal (ULN);
   b. Serum alanine aminotransferase > 3 × ULN;
   c. Total bilirubin > 2 × ULN;
   d. Estimated glomerular filtration rate by simplified 4-variable Modification of Diet in Renal Disease formula < 40 mL/min/1.73 m²;
   e. Absolute neutrophil count < 1,500/µL;
   f. Hemoglobin < 9 g/dL;
   g. Total white blood cell count < 2,500/µL;
   h. Platelet count < 150,000/µL;
   i. Absolute lymphocyte count < 800/µL.
17. Known history of allergy or reaction to any component of the KPL-404 or placebo formulation and/or other products in the same class.
18. History of cancer within the last 5 years from screening, except for basal and squamous cell carcinoma of the skin or in situ carcinoma of the cervix treated and considered cured.
19. History of clinically significant (per investigator's judgment) drug or alcohol abuse within the last 6 months.
20. History of any of the following cardiovascular conditions:
   a. Moderate to severe congestive heart failure (New York Heart Association class III or IV);
   b. Recent (within past 6 months) cerebrovascular accident, myocardial infarction, coronary stenting;
   c. Uncontrolled hypertension as defined by a confirmed systolic blood pressure > 160 mmHg or diastolic blood pressure > 100 mmHg.
21. Clinically relevant or significant electrocardiogram (ECG) abnormalities, including ECG with QT interval corrected for heart rate (QTc) > 500 msec.
22. Any condition that, in the opinion of the investigator, could interfere with evaluation of KPL-404 or interpretation of subject safety or confound the results of the study.

### TREATMENT OF SUBJECTS

### Description of Study Drug

**Table 4: Investigational Product**

| | **Investigational Product** | |
|---|---|---|
| **Product Name:** | **KPL-404** | **Placebo** |
| **Dosage Form:** | KPL-404 drug product is manufactured at 200 mg/mL as a sterile preservative-free solution in a single use vial for parenteral administration. | Placebo is a sterile preservative-free solution for parenteral administration with identical composition as the drug product, but without the protein. |
| **Unit Dose** | **Cohort 1:** 2 mg/kg KPL-404 q2wk | Placebo volume will be matched to KPL-404 at the corresponding dosage level. |
| | **Cohort 2:** 5 mg/kg KPL-404 q2wk | |
| | **Cohort 3:** 10 mg/kg KPL-404 q2wk | |
| | **Cohort 4:** 10 mg/kg KPL-404 SC q2wk; 5 mg/kg SC q2wk | |
| | **Cohort 5 (optional):** KPL-404 to be determined | |
| | **Cohort 6 (optional):** KPL-404 to be determined | |
| **Route of Administration** | Cohorts 1-4: SC | Cohorts 1-4: SC |
| | Cohorts 5-6: to be determined | Cohorts 5-6: to match KPL-404 |
| **Physical Description** | KPL-404 drug product is manufactured at 200 mg/mL as a sterile, preservative-free solution for parenteral administration. It is supplied as a single-use vial with a 1.0 mL extractable volume. | Placebo is a sterile preservative-free solution for parenteral administration with identical composition as the drug product, but without the protein. It is supplied as a single-use vial with 1.0 mL extractable volume. |

| | | |
|---|---|---|
| q2wk = every 2 weeks. | | |

### Randomization

All subjects who are eligible for study participation will be randomized on Day 1 prior to study drug administration. Each subject will be randomized to receive KPL-404 or placebo as per the randomization schedule. Randomization will be stratified by prior treatment with a bDMARD or JAKi.

For Cohorts 1-3, subjects will be randomized 3:1, KPL-404 to placebo.

The overall randomization ratio in Cohort 4 is 1:1:1 (5 mg/kg, 10 mg/kg, placebo). To maintain blinding given the differing volumes of the two active dose groups, two placebo volumes matched to the corresponding 5 mg/kg and 10 mg/kg volumes will be used, which leads to *a de facto* 2:2:1:1 randomization ratio with the final two numbers representing the two different volumes of placebo. Subjects from both placebo volumes will be pooled in all analyses. Pharmacists and investigators will only be unblinded to dose level (10 mg/kg vs 5 mg/kg) and remain blinded to the treatment (active treatment vs placebo) throughout.

### STUDY DRUG MATERIALS AND MANAGEMENT

### Study Drug

KPL-404 drug product is a monoclonal antibody consisting of 2 heavy chains and 2 light chains covalently linked by disulfide bridges (i.e., a heavy chain variable region having the amino acid sequence of SEQ ID NO: 9 and a light chain variable region having the amino acid sequence of SEQ ID NO: 10). It binds to CD40 with an affinity of 7.2 nM, and blocks CD154-mediated activation of B-cells.

Placebo includes the above excipients only but without the active ingredient.

### Administration

KPL-404 drug product and placebo will be administered subcutaneously. There is no restriction on fasting, water intake, or postures pre-/post dose.

### STUDY ASSESSMENTS

### Order of Assessments

The following assessments should be conducted in the order below as required at a pre-dose and post-dose time points:
1. Subject-reported outcomes
2. Tender joint count (TJC) 68/Swollen joint count (SJC) 66 and clinical assessments
3. 12-lead ECG
4. Chest X-ray (for screening only, if required)
5. Vital signs
6. PK/PD sampling
7. Blood sampling for safety, efficacy, and biomarker assessments
8. Biopsy samples (for subjects participating in the biopsy sub-study)
9. Study drug administration (if applicable)
10. Observational period: subjects will be observed for 60 minutes after dosing on Day 1 and 30 minutes after dosing through end of treatment.

### Efficacy Assessments

### TJC/SJC

The 68 joints will be examined and assessed as tender or not tender for TJC and 66 joints will be examined and assessed as swollen or not swollen for SJC. The ACR response criteria is based on TJC68/SJC66. The 68 joints (34 joints on each side of the subject's body) to be assessed and classified as tender or not tender include: 2 temporomandibular joints, 2 sternoclavicular joints, 2 acromioclavicular joints, 2 shoulder joints, 2 elbow joints, 2 wrist joints, 10 MCP joints, 2 interphalangeal joints of the thumb, 8 proximal interphalangeal joints of the hands, 8 distal interphalangeal joints of the hands, 2 hip joints, 2 knee joints, 2 ankle joints, 2 tarsus, 10 metatarsophalangeal joints of the feet, 2 great toes (first proximal interphalangeal joint of the feet), and 8 proximal interphalangeal joints of the feet. The 66 joints (33 joints on each side of the subject's body) to be assessed and classified as swollen or not swollen include all TJC68 joints except 2 hip joints.

The DAS28-CRP, CDAI, and SDAI are based on the TJC28/SJC28. The 28 joints will be examined and assessed as tender or not tender for TJC and as swollen or not swollen for SJC (Smolen 1995). The following 28 joints (14 joints on each side of the subject's body) will be assessed for tenderness and swelling: the 8 proximal interphalangeal joints of the fingers, the interphalangeal joints of the thumbs (n = 2), the 10 metacarpophalangeal joints plus the wrists (n = 2), elbows (n = 2), shoulders (n = 2), and knees (n = 2).

The TJC and SJC will be performed according to the Schedule of Activities in Table 3.

### Physician's Global Assessment

The Physician's Global Assessment (PhGA) of disease activity is a 100-mm visual analog scale (VAS) that asks the investigator to rate their subject's current disease activity on a scale of 0 mm (none) to 100 mm (extremely active). This PhGA will be used in the calculation of the CDAI, SDAI, and ACR20/50/70. These metrics are described, for example, in Arthritis Rheum. 15;57(2):193-202, 2007; Erratum in: Arthritis Rheum. 15;57(8):1574, 2007; and Smolen et al., Clin Exp Rheumatol. 23(5 Suppl 39):S100-8, 2005, the disclosures of each of which are hereby incorporated by reference in their entirety.

### Subject-Reported Outcomes

### Subject Global Assessment

The Subject Global Assessment (PGA) of disease activity is a measure of the subject's general health on a 100-mm visual analogue scale (VAS) from 0 mm = best to 100 mm = worst. This PGA will be used in the calculation of the DAS28-CRP, CDAI, SDAI, and ACR20/50/70 (see, e.g., Van Riel, Clin Exp Rheumatol. 34(5 Suppl 101):S40-S44, 2006, the disclosure of which is hereby incorporated by reference in its entirety).

### Pain Visual Analog Scale

Subjects will be asked to place a line perpendicular to the VAS line at the point that represents the intensity of their most severe arthritis pain using a scale from 0 to 100, with 0 indicating no pain and 100 indicating the worst pain. This pain VAS will be used at baseline and at scheduled time points through EOS.

### Health Assessment Questionnaire-Disability Index

The Health Assessment Questionnaire-Disability Index (HAQ-DI) is composed of 8 categories, reviewing a total of 20 specific functions evaluating subject difficulty with activities of daily living over the past week. Categories include dressing and grooming, arising, eating, walking, hygiene, reaching, gripping, and errands and chores. Also identified are specific aids or devices utilized for assistance, as well as help needed from another person (aides/help).

### SF-36 Physical Health Composite Score

SF-36 measures 8 domains of health-related quality of life within 8 scales: 1) limitations in physical activities because of health problems; 2) limitations in social activities because of physical or emotional problems; 3) limitations in usual role activities because of physical health problems; 4) bodily pain; 5) general mental health (psychological distress and well-being); 6) limitations in usual role activities because of emotional problems; 7) vitality (energy and fatigue); and 8) general health perceptions. The domains are used to calculate composite scores - physical health composite score (PCS) and mental health composite score (MCS), as well as SF-36 total composite score (TCS). The scores for the SF-36 are based on a 0 to 100 scale; zero represents the lowest possible score and 100 represents the highest possible score, with higher score indicating a better health state.

### Synovial Biopsy Substudy

A synovial tissue biopsy is optional and will be conducted in subjects consenting to the biopsy substudy to measure biomarkers in synovial tissue. The investigator will have the option to collect biopsy per the Schedule of Activities in Table 3. Details of synovial biopsy collection will be provided in a separate manual.

### Pharmacokinetic Assessments

Pharmacokinetic (PK) blood samples will be collected by venipuncture or cannulation at the times indicated in the Schedule of Activities in Table 3. Procedures for collection and processing of PK blood samples will be detailed in the Laboratory Manual.

Serum concentrations of KPL-404 will be determined using a validated analytical procedure. Specifics of the analytical method will be provided in a separate report.

### Immunogenicity Assessments

All subjects will be monitored for the development of a humoral immune response to KPL-404. Blood samples will be assessed in all subjects for the presence of anti-KPL-404 antibodies (ADA) by a validated screening assay at baseline and per the Schedule of Activities in Table 3.

The effect of ADA on the PK properties of KPL-404 in ADA positive subjects may be assessed.

### Pharmacodynamic Biomarker Assessments

Circulating biomarker samples will be collected by venipuncture or cannulation at the times indicated in the Schedule of Activities in Table 3. Procedures for collection and processing of biomarker blood samples will be detailed in the Laboratory Manual.

### STATISTICAL ANALYSES

Statistics to be reported are outlined in this section. Analysis plans will be written and approved prior to performing the first interim analysis. All statistical analysis and reporting will be done using SAS 9.4 or higher.

Descriptive statistics and changes from baseline, as applicable, will be calculated for safety parameters.

### Sample Size Justification

The sample size for Cohorts 1, 2, and 3 are based on safety and PK considerations. The sample size for Cohort 4 is based on a 2-sample t-test assuming an improvement in DAS28-CRP change from baseline for the KPL-404 being 1.2 points better than placebo (standard deviation = 1.3). Enrollment of 21 subjects in each of the 3 treatment arms (KPL-404 10 mg/kg, KPL-404 5 mg/kg, and placebo) allows 90% power for the treatment comparison with 2-sided 0.1 type 1 error if all subjects complete 12 weeks of DAS28-CRP assessments. In order to account for an anticipated discontinuation rate of 16%, each dose level will be achieved by enrolling 20 subjects per arm in Cohort 4 in addition to the 6 subjects per arm enrolled in Cohorts 1, 2, and 3. Since Cohorts 1, 2, and 3 would have already enrolled 6 subjects for each of the 3 treatment arms, up to an additional 20 subjects per arm and for a total of up to 60 subjects are required to enroll under Cohort 4.

### Analysis of Efficacy: Efficacy Endpoints

### DAS28-CRP

The primary endpoint for Cohort 4 and a secondary endpoint for Cohorts 1-3 is change from baseline in DAS28-CRP at Week 12. The DAS28-CRP score is derived from the number of swollen joints (out of 28 assessed), number of tender joints (out of 28 assessed), CRP level, and PGA score.

### Low Disease Activity and Complete Response

Low disease activity is defined as one of the following:
• DAS28-CRP ≤ 3.2
• CDAI ≤ 10.0
• SDAI ≤ 11.0

Complete response is defined as one of the following:
• DAS28-CRP < 2.6
• CDAI ≤ 2.8
• SDAI ≤ 3.3

Clinical disease activity index (CDAI) is derived from the TJC/SJC of 28 joints, the PGA score, and the PhGA score. The SDAI is derived from the CDAI plus DAS28-CRP levels.

### ACR20/50/70

The ACR20/50/70 corresponds to at least a 20%, 50%, or 70% improvement in the number of tender (TJC68) and swollen joints (SJC66) from baseline and a corresponding improvement in 3 of the following 5 criteria:
• PGA
• Pain VAS
• PhGA
• HAQ-DI
• CRP level

### Analysis Methods

For efficacy analysis, a 2-sided type 1 error rate of 0.1 will be used if applicable.

For Cohorts 1, 2, and 3, only descriptive statistics and listings will be produced for all efficacy parameters. No formal statistical testing for efficacy will be performed.

For Cohort 4 (if appropriate, pooled with Cohort 1, 2, and 3) and for additional Cohorts, if pursued, analysis of covariance (ANCOVA) models will be used to compare the change from baseline in DAS28-CRP at Week 12 for each of the KPL-404 dose groups and placebo. Baseline and randomization stratification factor will be included as covariates in ANCOVA models. No testing between the two KPL-404 dose groups will be conducted. ACR response rates at Week 12 between each of the KPL-404 dose groups and placebo will be compared using Fisher's exact test. In addition, logistic regression models may be produced including key baseline characteristics as covariates in addition to treatment.

Baseline is defined as the last pre-dose value on or before Day 1.

### Analysis of Pharmacokinetics

Descriptive statistics (arithmetic mean, standard deviation, minimum, median, maximum, geometric mean, and geometric coefficient of variation, as appropriate) will be listed and summarized for serum concentrations of KPL-404 and PK parameters. There data are available, KPL-404 dose proportionality will be examined between the dose groups. The AUC_{0-∞}, AUC₀₋ₜ, and Cₘₐₓ estimates will be tested for dose proportionality using a power model approach or analysis of variance (ANOVA) model as appropriate. There data are available, exposure of KPL-404 administered by SC injection will be compared across dose levels. Log-transformed AUC_{0-∞} and AUC₀₋ₜ estimates will be analyzed using an ANOVA model with group as a fixed effect. Other analytical tests may be employed depending on the characteristics of the dataset. Additional details regarding analyses will be provided in the SAP for PK analysis. Additional exploratory and post hoc analyses of the data may be conducted as deemed appropriate.

### Analysis of Immunogenicity

Serum ADA titers will be listed and summarized. The correlation between serum anti-KPL-404 antibodies and KPL-404 concentrations and efficacy may also be evaluated as appropriate. No formal hypothesis testing is planned.

### Phase 2 Modification

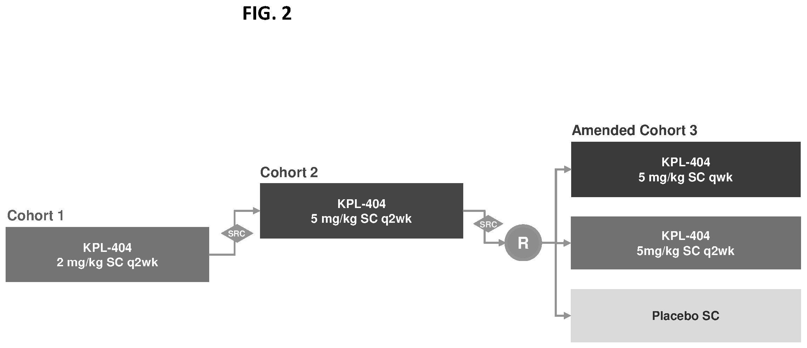

A modified Phase 2 Trial scheme is shown in FIG. 2. The 12-week study was designed to provide PK data and early signal of efficacy with chronic administration. For Cohorts 1 and 2, each cohort will sequentially randomize 8 patients and Cohort 3 will randomize up to 75 patients. In Cohort 1, subjects receive 2 mg/kg SC q2wk. In Cohort 2, subjects receive 5 mg/kg SC q2wk. Cohort 3 subjects receive 5 mg/kg SC qwk, 5 mg/kg SC q2wk, or placebo SC. Cohorts 4-6 will not be used.

The patient population includes those with active RA who have an inadequate response to or are intolerant to a Janus kinase inhibitor (JAKi) or at least one biologic disease-modifying anti-rheumatic drug (bDMARD). Subjects who have failed both bDMARD and JAKi are excluded from the study (for ease of study readout; such subjects would be expected to respond to treatment with the anti-CD40 antibody).

Disease criteria include six or more swollen joints and ≥ 6 tender joints at screening and baseline line visits; levels of high sensitivity C-reactive protein ≥ 5 mg/L; seropositivity for serum RF and/or ACPA at screening.

For Cohorts 1 and 2 (8 randomized patients), primary endpoints include Incidence of treatment-emergent adverse events (TEAEs); and Pharmacokinetics (Cmax, AUC(0-t)); Secondary Endpoints include Change from baseline in DAS28-CRP at Week 12.

For amended Cohort 3, the cohort will randomize up to 75 patients. The primary endpoints include change from baseline in DAS28-CRP at Week 12. Secondary endpoints include incidence of treatment-emergent adverse events (TEAEs) and pharmacokinetics (Cmax, AUC(0-t)). Additional SRC was added for enhanced safety monitoring during trial.

### Example 3

### Treatment of Rheumatoid Arthritis

A male subject weighing 80 kg with rheumatoid arthritis can be treated with the KPL-404 antibody according to the methods described herein. The subject is classified as bDMARDs-inadequate response (bDMARD-IR) or JAKi-inadequate response (JAKi-IR) due to intolerance or toxicity, regardless of treatment duration. The antibody is formulated at 200 mg/mL at a dosage of 5 mg/kg every other week. The subject is characterized using a pain visual analog scale (VAS) and indicates a pain score at baseline of 90 (maximum of 100 indicates the worst pain). The subject is also assessed using an SF-36 physical health composite score and reports a baseline score of 10 (minimum 0 indicates poor health). The subject can be administered a 2.0 mL subcutaneous injection of the formulation once every two weeks for a treatment regimen of 12 weeks. Following 12 weeks of treatment, the subject's ACR50 score, ACR70 score, DAS28-CRP score, pain VAS score can be assessed for improvement (e.g., a decrease to a VAS score of about 40 (minimum of 0 indicates no pain)), and his SF-36 score can be assessed for improvement (e.g., an increase to a score of about 60 (maximum 100 indicates good health)). The treatment is maintained for an additional 12 weeks, and the subject's ACR50, ACR70, DAS28-CRP, VAS and SF-36 scores can be assessed for stability or further improvement.

### Other Embodiments

While specific aspects of the invention have been described and illustrated, such aspects should be considered illustrative of the invention only and not as limiting the invention as construed in accordance with the accompanying claims.

All publications and patent applications cited in this specification are herein incorporated by reference in their entirety for all purposes as if each individual publication or patent application were specifically and individually indicated to be incorporated by reference for all purposes. Although the foregoing invention has been described in some detail by way of illustration and example for purposes of clarity of understanding, it will be readily apparent to one of ordinary skill in the art in light of the teachings of this invention that certain changes and modifications can be made thereto without departing from the spirit or scope of the appended claims.

The following pages 44-47 contain further embodiments.
1. A method of treating rheumatoid arthritis comprising administering to a human subject by infusion or by intravenous or subcutaneous administration a pharmaceutical composition comprising a humanized anti-CD40 antibody or antigen-binding fragment thereof comprising a heavy chain variable region comprising the amino acid sequence set forth in SEQ ID NO: 9, and a light chain variable region comprising the amino acid sequence set forth in SEQ ID NO: 10, wherein the antibody or antigen-binding fragment thereof is administered at a dosage of from about 2 mg/kg to about 10 mg/kg at least once every two weeks or every month.
2. The method of item 1, wherein the pharmaceutical composition comprises the antibody or antigen-binding fragment thereof at a concentration of from about 50 mg/mL to about 300 mg/mL.
3. The method of item 2, wherein the pharmaceutical composition comprises the antibody or antigen-binding fragment thereof at a concentration of about 200 mg/mL.
4. The method of any one of items 1-3, wherein about 0.5 mL to about 5.0 mL of the pharmaceutical composition is administered to the subject.
5. The method of item 4, wherein a volume of about 2.0 mL of the pharmaceutical composition is administered to the subject.
6. The method of any one of items 1-5, wherein the antibody or antigen-binding fragment thereof is administered a dosage of about 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 6 mg/kg, 7 mg/kg, 8 mg/kg, 9 mg/kg, or 10 mg/kg.
7. The method of item 6, wherein the antibody or antigen-binding fragment thereof is administered intravenously at a dosage of about 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 6 mg/kg, 7 mg/kg, 8 mg/kg, 9 mg/kg, or 10 mg/kg.
8. The method of item 6, wherein the antibody or antigen-binding fragment thereof is administered subcutaneously at a dosage of about 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 6 mg/kg, 7 mg/kg, 8 mg/kg, 9 mg/kg, or 10 mg/kg.
9. The method of any one of items 1-8, wherein the composition is administered during a treatment regimen of at least one week, two weeks, three weeks, one month, two months, three months, four months, five months, six months, one year, or longer, or until clinical remission is achieved or a minimum low disease activity is achieved.
10. The method of any one of items 1-9, wherein the antibody or antigen-binding fragment thereof is administered subcutaneously at a concentration of about 2 mg/kg, about 5 mg/kg, or about 10 mg/kg once every week or every two weeks.
11. The method of item 10, wherein the composition is administered during a treatment regimen of about 12 weeks or longer, or until clinical remission is achieved or a minimum low disease activity is achieved.
12. The method of any one of items 1-11, further comprising administering a second agent.
13. The method of item 12, wherein the second agent is an immunosuppressant, a Janus kinase (JAK) inhibitor, or a disease modifying anti-rheumatic drug (DMARD).
14. The method of item 13, wherein the immunosuppressant is a calcineurin inhibitor, tacrolimus, an mTor inhibitor, fingolimod, myriocin, alemtuzumab, rituximab, an anti-CD4 monoclonal antibody, an anti-LFA1 monoclonal antibody, an anti-LFA3 monoclonal antibody, an anti-CD45 antibody, an anti-CD19 antibody, monabatacept, belatacept, indolyl¬ASC; azathioprine, lymphocyte immune globulin and anti-thymocyte globulin [equine], mycophenolate mofetil, mycophenolate sodium, daclizumab, basiliximab, cyclophosphamide, prednisone, prednisolone, leflunomide, FK778, FK779, 15-deoxyspergualin, busulfan, fludarabine, methotrexate, 6-mercaptopurine, 15-deoxyspergualin, LF15-0195, bredinin, brequinar, or muromonab-CD3.
15. The method of item 13, wherein the JAK inhibitor is, ruxolitinib, tofacitinib, oclacitinib, baricitinib, peficitinib, fedratinib, upadacitinib, filgotinib, or delgocitinib,
16. The method of item 13, wherein the DMARD is abatacept, adalimumab, anakinra, apremilast, azathioprine, baricitinib, certolizumab pegol, chloroquine, ciclosporin (Cyclosporin A), D-penicillamine, etanercept, filgotinib, golimumab, gold salts (e.g., sodium aurothiomalate or auranofin), hydroxychloroquine, infliximab, leflunomide, methotrexate, minocycline, rituximab, sarilumab, secukinumab, sulfasalazine, tocilizumab, tofacitinib, or ustekinumab.
17. The method of item 13, wherein the DMARD is a conventional synthetic DMARD.
18. The method of any one of items 1-17, wherein the subject has had an inadequate response, or is intolerant, to a JAK inhibitor or a DMARD.
19. The method of any one of items 1-18, wherein a 20% improvement in the American College of Rheumatology core set disease index (ACR20) is achieved after treatment.
20. The method of any one of items 1-18, wherein a 50% improvement in the American College of Rheumatology core set disease index (ACR50) is achieved after treatment.
21. The method of item 19, wherein the ACR20 is achieved following a treatment regimen of about 1 week.
22. The method of item 19, wherein the ACR20 is achieved following a treatment regimen of about 24 weeks.
23. The method of item 19, wherein the ACR20 is achieved following a treatment regimen of about 52 weeks.
24. The method of item 20, wherein the ACR50 is achieved following a treatment regimen of about 12 weeks.
25. The method of item 20, wherein the ACR50 is achieved following a treatment regimen of about 24 weeks.
26. The method of item 20, wherein the ACR50 is achieved following a treatment regimen of about 52 weeks.
27. The method of any one of items 1-18, wherein the disease activity score of 28 joints using C-reactive protein (DAS28-CRP) is achieved after treatment.
28. The method of item 27, wherein the DAS28-CRP is achieved following a treatment regimen of about 12 weeks.
29. The method of item 27, wherein the DAS28-CRP is achieved following a treatment regimen of about 24 weeks.
30. The method of item 27, wherein the DAS28-CRP is achieved following a treatment regimen of about 52 weeks.
31. The method of any one of items 1-18, wherein after treatment the subject achieves an improvement in a disease activity determined by a criteria selected from the group consisting of ACR20, ACR50, ACR70, DAS28, DAS28-CRP, Physician's Global Assessment (PhGA) of disease activity, Health Assessment Questionnaire-Disability Index (HAQ-DI), Short Form Health survey (SF-36) in the physical health composite score (PCS) and mental health composite score (MCS), SF-36 total composite score (TCS), Clinical Disease Activity Index (CDAI), Simple Disease Activity Index (SDAI), and a pain Visual Analog Scale (VAS), and combinations thereof.

## Claims

1. A pharmaceutical composition comprising a humanized anti-CD40 antibody or antigen-binding fragment thereof for use in a method of treating rheumatoid arthritis, wherein the antibody or antigen-binding fragment thereof comprises a heavy chain variable region comprising the amino acid sequence set forth in SEQ ID NO: 9, and a light chain variable region comprising the amino acid sequence set forth in SEQ ID NO: 10, wherein the antibody or antigen-binding fragment thereof is administered to a human subject intravenously or subcutaneously at a dosage of from about 2 mg/kg to about 10 mg/kg at least once every week, once every two weeks or once per month.

2. The pharmaceutical composition for use of claim 1, wherein the pharmaceutical composition comprises the antibody or antigen-binding fragment thereof at a concentration of from about 50 mg/mL to about 300 mg/mL, wherein, optionally, the concentration is about 200 mg/mL.

3. The pharmaceutical composition for use of claim 1 or 2, wherein about 0.5 mL to about 5.0 mL of the pharmaceutical composition is administered to the subject, wherein, optionally, the volume is about 2.0 mL of the pharmaceutical composition.

4. The pharmaceutical composition for use of any one of claims 1-3, wherein the antibody or antigen-binding fragment thereof is administered a dosage of about 5 mg/kg, 1 mg/kg, 1.5 mg/kg, 2 mg/kg, 2.5 mg/kg, 3 mg/kg, 3.5 mg/kg, 4 mg/kg, 4.5 mg/kg, 6 mg/kg, 7 mg/kg, 8 mg/kg, 9 mg/kg, or 10 mg/kg.

5. The pharmaceutical composition for use of claim 4, wherein the antibody or antigen-binding fragment thereof is administered intravenously at a dosage of about 5 mg/kg or 6 mg/kg.

6. The pharmaceutical composition for use of any one of claims 1-5, wherein the composition is administered during a treatment regimen of at least one week, two weeks, three weeks, one month, two months, three months, four months, five months, six months, one year, or longer, or until clinical remission is achieved or a minimum low disease activity is achieved.

7. The pharmaceutical composition for use of any one of claims 1-6, wherein the antibody or antigen-binding fragment thereof is administered subcutaneously at a concentration of about 5 mg/kg or about 6 mg/kg once every week, once every two weeks or once per month.

8. The pharmaceutical composition for use of claim 7, wherein the composition is administered during a treatment regimen of about 12 weeks or longer, or until clinical remission is achieved or a minimum low disease activity is achieved.

9. The pharmaceutical composition for use of any one of claims 1-8, the method further comprising administering a second agent.

10. The pharmaceutical composition for use of claim 9, wherein the second agent is an immunosuppressant, a Janus kinase (JAK) inhibitor, or a disease modifying anti-rheumatic drug (DMARD).

11. The pharmaceutical composition for use of claim 10, wherein:
a) the immunosuppressant is a calcineurin inhibitor, tacrolimus, an mTor inhibitor, fingolimod, myriocin, alemtuzumab, rituximab, an anti-CD4 monoclonal antibody, an anti-LFA1 monoclonal antibody, an anti-LFA3 monoclonal antibody, an anti-CD45 antibody, an anti-CD19 antibody, monabatacept, belatacept, indolyl-ASC; azathioprine, lymphocyte immune globulin and anti-thymocyte globulin [equine], mycophenolate mofetil, mycophenolate sodium, daclizumab, basiliximab, cyclophosphamide, prednisone, prednisolone, leflunomide, FK778, FK779, 15-deoxyspergualin, busulfan, fludarabine, methotrexate, 6-mercaptopurine, 15-deoxyspergualin, LF15-0195, bredinin, brequinar, or muromonab-CD3;
b) the JAK inhibitor is, ruxolitinib, tofacitinib, oclacitinib, baricitinib, peficitinib, fedratinib, upadacitinib, filgotinib, or delgocitinib;
c) the DMARD is abatacept, adalimumab, anakinra, apremilast, azathioprine, baricitinib, certolizumab pegol, chloroquine, ciclosporin (Cyclosporin A), D-penicillamine, etanercept, filgotinib, golimumab, gold salts (e.g., sodium aurothiomalate or auranofin), hydroxychloroquine, infliximab, leflunomide, methotrexate, minocycline, rituximab, sarilumab, secukinumab, sulfasalazine, tocilizumab, tofacitinib, or Ustekinumab; and/or
d) the DMARD is a conventional synthetic DMARD.

12. The pharmaceutical composition for use of any one of claims 1-11, wherein the subject has had an inadequate response, or is intolerant, to a JAK inhibitor or a DMARD.

13. The pharmaceutical composition for use of any one of claims 1-12, wherein a 20% improvement in the American College of Rheumatology core set disease index (ACR20) is achieved after treatment or wherein a 50% improvement in the American College of Rheumatology core set disease index (ACR50) is achieved after treatment.

14. The pharmaceutical composition for use of any one of claims 1-13, wherein the disease activity score of 28 joints using C-reactive protein (DAS28-CRP) is achieved after treatment, wherein, optionally, the DAS28-CRP is achieved following a treatment regimen of about 12, 24, or 52 weeks.

15. The pharmaceutical composition for use of any one of claims 1-14, wherein after treatment the subject achieves an improvement in a disease activity determined by a criteria selected from the group consisting of ACR20, ACR50, ACR70, DAS28, DAS28-CRP, Physician's Global Assessment (PhGA) of disease activity, Health Assessment Questionnaire-Disability Index (HAQ-DI), Short Form Health survey (SF-36) in the physical health composite score (PCS) and mental health composite score (MCS), SF-36 total composite score (TCS), Clinical Disease Activity Index (CDAI), Simple Disease Activity Index (SDAI), and a pain Visual Analog Scale (VAS), and combinations thereof.
